# EUROPÄISCHE PATENTANMELDUNG

(11) **EP 2 014 275 A2**
(43) Veröffentlichungstag der Anmeldung: **14.01.2009**
(21) Anmeldenummer: 08003853.2
(22) Anmeldetag: 01.03.2008
(51) Int. Cl.: A61K 8/33, A61Q 5/10

(54) **Kit zur Bereitstellung von lagerstabilen Formulierungen**

(30) Priorität: 15.06.2007 DE 102007028266
(71) Anmelder: Henkel AG & Co. KGaA, 40589 Düsseldorf (DE)
(72) Erfinder: Oberkobusch, Doris, 40591 Düsseldorf (DE); Groß, Wibke, 40549 Düsseldorf (DE); Stephan, Laura, 40589 Düsseldorf (DE)

(57) **Zusammenfassung**

Es wird ein Kit zum Färben von keratinhaltigen Fasern beschrieben, welcher mindestens zwei spezielle, besonders lagerstabile kosmetische Mittel A und B umfasst, ferner die Verwendung dieses Kits zum Färben von Haaren und ein entsprechendes Färbeverfahren. Das Mittel A enthält in einem kosmetischen Träger mindestens eine reaktive Carbonylverbindung und weist einen pH-Wert von 2 bis 5 auf. Das Mittel B enthält in einem kosmetischen Träger mindestens eine CH-acide Verbindung ausgewählt aus mindestens einer Verbindung der Formel (CH-1) und/oder (CH-2). 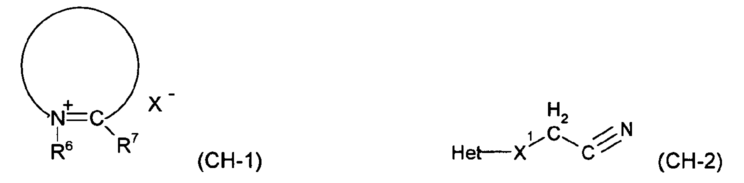 worin
• R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R^{I}R^{II}N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
• R⁷ steht für eine (C₁ bis C₆)-Alkylgruppe, insbesondere für eine Methylgruppe,
• X- steht für ein physiologisch verträgliches Anion,
• der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
• Het steht für einen gegebenenfalls substituierten Heteroaromaten,
• X¹ steht für eine direkte Bindung oder eine Carbonylgruppe
und weist einen pH-Wert von 0,5 bis 2,5 auf.

## Beschreibung

Die vorliegende Erfindung betrifft ein Kit zum Färben von keratinhaltigen Fasern, insbesondere menschlichen Haaren, welches mindestens zwei spezielle kosmetische Mittel A und B umfasst, ferner die Verwendung dieses Kits zum Färben von Haaren und ein entsprechendes Färbeverfahren. Das Mittel A enthält hierbei in einem kosmetischen Träger mindestens eine reaktive Carbonylverbindung und weist einen pH-Wert von 2 bis 5 auf. Das Mittel B enthält in einem kosmetischen Träger mindestens eine spezielle CH-acide Verbindung und besitzt einen pH-Wert zwischen 0,5 und 2,5.

Zum Färben von Haaren sind dem Fachmann verschiedene Färbesysteme bekannt, welche sich in der Haltbarkeit der Färbung auf dem Haar und dem Schädigungsgrad, welchen das Haar während des Haarfärbeprozesses erleidet, stark unterscheiden.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 4-Amino-3-methylphenol, 2-Aminomethyl-4-aminophenol, 4,5-Diamino-1-(2-hydroxyethyl)pyrazol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N, N'-bis(4-aminophenyl)-diamino-propan-2-ol.

Als Kupplerkomponenten werden in der Regel m-Phenylendiaminderivate, Naphthole, Pyridinderivate, Resorcin und Resorcinderivate, Pyrazolone und m-Aminophenole verwendet. Als Kupplersubstanzen eignen sich insbesondere 1-Naphthol, 1,5-Dihydroxynaphthalin, 2,7-Dihydroxynaphthalin, 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazol-5-on, 2,4-Di-chlor-3-aminophenol, 1,3-Bis-(2,4-diaminophenoxy)-propan, 2-Amino-3-hydroxypyridin, 4-Chlorresorcin, 2-Chlor-6-methyl-3-aminophenol, 2-Methylresorcin, 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Eine weitere Möglichkeit zur Farbveränderung bietet die Verwendung von Färbemitteln, welche sogenannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind.

Das mit der schonenden Oxofärbung erzielbare Nuancenspektrum ist sehr breit und die erhaltene Färbung weist oftmals eine akzeptable Brillanz und Farbtiefe auf. Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Da bei der Oxofärbung auch ohne den Einsatz von Oxidationsmitteln wie beispielsweise Wasserstoffperoxid leuchtende Farben mit guter Haltbarkeit auf dem Haar erzielt werden können, ist diese Färbemethode mit einer geringen Haarschädigung verbunden und vor diesem Hintergrund für den Verbraucher von besonderem Interesse.

Die technische Realisierung der Oxofärbung wird beispielsweise in WO 2004/022016 A1 und WO 2005/120445 A2 offenbart.

In DE 10022743 A1 wird ein Mittel zum Färben von Haaren beschrieben, welches durch Vermischen einer ein Enamin oder dessen Säureadditionssalz enthaltenden Komponente (A1) und einer eine Carbonylverbindung und ein primäres Amin enthaltenden Komponente (A2) hergestellt wird. Hierbei weist die Komponente A1 einen sauren pH-Wert auf, die Komponente A2 besitzt jedoch einen alkalischen pH-Wert.

Von der Konfektionierung und Verpackung des Färbemittels über den Verkauf bis zur Anwendung beim Verbraucher können Zeiträume von mehreren Monaten bis sogar einigen Jahren vergehen. Verwendet der Verbraucher ein Haarfärbemittel, welches diese langen Lagerungszeiträume durchlaufen hat, so wünscht er sich doch ein brillantes und intensives Farbergebnis. Die Bereitstellung des Färbemittels in einer stabilen Lagerform ist daher eine der zentralen Herausforderungen bei der Konfektionierung.

Sowohl die reaktiven Carbonylverbindungen der Komponente Oxo 1 als auch die CH-aciden Verbindungen der Komponente Oxo2 besitzen in Lösung nur eine begrenzte Stabilität, so dass sich die Verbindungen oftmals im Laufe einiger Wochen bis Monate zersetzen. Der hieraus resultierende Abfall der Farbintensität bei Anwendung des Färbemittels wird vom Verbraucher nicht akzeptiert.

Aufgabe der vorliegenden Erfindung ist es daher, im Rahmen der Oxofärbung ein Kit zum Färben von Haaren zur Verfügung zu stellen, wobei sowohl die Carbonylverbindung (Oxo 1) als auch die CH-acide Verbindung (Oxo 2) stabilisiert in der Formulierung vorliegen, so dass auch nach langen Lagerzeiträumen Färbungen von hoher Farbintensität erhalten werden können.

Überraschenderweise konnte nun gefunden werden, dass eine getrennte Konfektionierung der Carbonylverbindungen der Komponente Oxo 1 und der CH-aciden Verbindungen der Komponente Oxo 2 in zwei kosmetischen Formulierungen, wobei der pH-Wert der beiden Formulierungen jeweils in einem bestimmten sauren pH-Bereich liegt, die Lagerstabilität des Färbemittels in nicht vorhersehbarer Weise stark erhöht.

Ein erster Gegenstand der Erfindung ist daher ein Kit zum Färben von keratinhaltigen Fasern, insbesondere menschlichen Haaren, umfassend kosmetische Mittel A und B, wobei
- das Mittel A in einem kosmetischen Träger mindestens eine reaktive Carbonylverbindung enthält und einen pH-Wert von 2 bis 5 aufweist und
- das Mittel B in einem kosmetischen Träger mindestens eine CH-acide Verbindung ausgewählt aus Verbindungen der Formel (CH-1) und/oder Verbindungen der Formel (CH-2) enthält 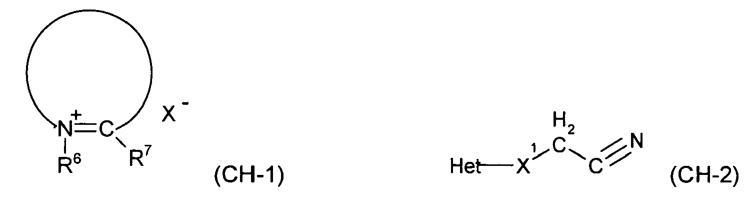
worin
- R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R^{I}R^{II}N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R' und R" gemeinsam mit dem Stickstoffatom einen 5-, 6- oder 7-gliedrigen Ring bilden können und m steht für eine Zahl 2, 3, 4, 5 oder 6,
- R⁷ steht für eine (C₁ bis C₆)-Alkylgruppe, insbesondere für eine Methylgruppe,
- X- steht für ein physiologisch verträgliches Anion,
- der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
- Het steht für einen gegebenenfalls substituierten Heteroaromaten,
- X¹ steht für eine direkte Bindung oder eine Carbonylgruppe und das Mittel B einen pH-Wert von 0,5 bis 2,5 aufweist.

Es wurde demzufolge gefunden, dass die Konfektionierung von reaktiven Carbonylverbindungen in einem kosmetischen Träger, wobei der pH-Wert dieses Mittels zwischen 2 und 5 liegt, die Lagerstabilität der Carbonylverbindungen in beträchtlichem Maße erhöht.

Auch die Einarbeitung der speziellen CH-aciden Verbindungen in einen kosmetischen Träger, wobei der pH-Wert dieses Mittels zwischen 0,5 und 2,5 liegt, erhöht die Stabilität der Formulierung in dem Maße, dass es auch bei einer Lagerung über mehrer Monate zu keinem signifikanten Abbau des Gehaltes an CH-acider Verbindung kommt.

Besonders lagerstabile Formulierungen können erhalten werden, wenn das Mittel A einen pH-Wert zwischen 3 und 4,5 aufweist, daher ist dieser pH-Bereich bevorzugt.

Desweiteren ist es bevorzugt, wenn das Mittel B einen pH-Wert von 1 bis 2 und insbesondere bevorzugt einen pH-Wert von 1,1 bis 1,9 aufweist.

Bei den pH-Werten im Sinne der vorliegenden Erfindung handelt es sich um pH-Werte, die bei einer Temperatur von 22 °C gemessen wurden.

Die Einstellung des pH-Wertes in den Mitteln A und/oder B kann mit Hilfe einer organischen oder anorganischen Säure wie beispielsweise Salzsäure, Schwefelsäure, Bromwasserstoffsäure, Phosphorsäure, Essigsäure, Weinsäure, Zitronensäure, Milchsäure, Äpfelsäure oder Glykolsäure erfolgen.

In diesem Zusammenhang ist die Einstellung der erfindungsgemäßen pH-Werte mit Hilfe von Salzsäure, Weinsäure, Zitronensäure, Äpfelsäure oder Milchsäure besonders bevorzugt.

Für die Anwendung des erfindungsgemäßen Kits können die Mittel A und B kurz vor dem Aufbringen auf die Haarfaser innig miteinander vermischt werden. Zur weiteren Verbesserung des Färbeergebnisses kann es jedoch vorteilhaft sein, die Färbung selbst in einem alkalischen pH-Bereich durchzuführen.

Daher kann in einer besonderen Ausführungsform der erfindungsgemäße Kit zusätzlich ein drittes Mittel C umfassen, welches in einem kosmetischen Träger mindestens ein Alkalisierungsmittel enthält und einen pH-Wert größer als 7 aufweist.

Wird die Färbung in einem alkalischen pH-Bereich durchgeführt, so werden beispielsweise vor dem Aufbringen auf die Haarfaser die Komponenten A, B und C sorgfältig miteinander vermengt.

Bei dem in Mittel C in einem kosmetischen Träger enthaltenen Alkalisierungsmittel können bevorzugt Alkalisierungsmittel ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus Ammoniak, 2-Aminoethanol, 2-Amino-2-methylpropan-1-ol, 2-Amino-2-methyl-propan-1,3-diol, Kaliumhydroxid, Natriumhydroxid, L-Arginin, D-Arginin, DL-Arginin, N-Methylglucamin, Morpholin, Imidazol und Harnstoff.

Desweiteren können auch auf einen pH-Wert größer als 7 eingestellte Puffersysteme in Mittel C eingesetzt werden.

Als pH-Puffersystem werden erfindungsgemäß solche chemische Verbindungen bzw. eine Kombination aus chemischen Verbindungen angesehen, die in einer Lösung bewirken, dass sich der pH-Wert der Lösung bei Zugabe einer kleinen Menge Säure oder Lauge zu einem Volumen des kosmetischen Trägers nur geringfügig ändert. Diese Änderung ist weniger ausgeprägt, als dies bei einer Zugabe der gleichen Menge an Säure oder Lauge zu einem gleichen Volumen des kosmetischen Trägers ohne pH-Puffersystem der Fall ist.

Solche pH-Puffersysteme sind bevorzugt ausgewählt aus mindestens einem Vertreter aus der Gruppe, die gebildet wird aus Hydrogencarbonat/Carbonat, Genußsäure (insbesondere Citronensäure)/Monohydrogenphosphat, Genußsäure (insbesondere Citronensäure)/Dihydrogenphosphat, Tris(hydroxymethyl)aminomethan/Maleinsäure/NaOH, Tris(hydroxymethyl)aminomethan/Maleinsäure/KOH, Tris(hydroxymethyl)aminomethan/HCl, Monohydrogenphosphat/Dihydrogenphosphat, Dihydrogenphosphat/NaOH, Dihydrogenphosphat/KOH, H₃BO₃/KCI/NaOH, H₃BO₃/KCI/KOH, Borat/HCl, Borat/Halogenid (insbesondere Chlorid wie Kaliumchlorid)/NaOH, Borat/Halogenid (insbesondere Chlorid wie Kaliumchlorid)/KOH, Puffersystem nach Theorell und Stenhagen, Puffersystem nach Mcllvine, Glycin/NaOH und Glycin/KOH. Besonders bevorzugte pH-Puffersysteme werden ausgewählt aus mindestens einem Vertreter aus der Gruppe, die gebildet wird, aus Tris(hydroxymethyl)aminomethan/Maleinsäure/NaOH, Tris(hydroxymethyl)amino-methan/Maleinsäure/KOH, Tris(hydroxymethyl)aminomethan/HCl, Borat/HCl und H₃BO₃/KCl/NaOH.

Die mit dem Schrägstrich gekennzeichneten pH-Puffersysteme aus obiger Liste stellen Gemische dieser durch den Schrägstrich getrennten Verbindungen dar. Die in der Liste angegebenen anionischen Verbindungen werden in Form deren Salze mit einem korrespondierenden ein- oder mehrwertigen Kation eingesetzt. Bevorzugte Kationen sind Alkalimetallkationen (insbesondere Natrium oder Kalium) und Ammoniumionen.
Erfindungsgemäß in den Puffersystemen verwendbare Genußsäuren sind beispielsweise Citronensäure, Weinsäure oder Äpfelsäure bzw. deren Gemische.

Das pH-Puffersystem ist bevorzugt in einer Menge von 0,1 bis 10,0 Gew.-%, besonders bevorzugt von 0,3 bis 5,0 Gew.-%, ganz besonders bevorzugt von 0,5 bis 3,0 Gew.-%, jeweils bezogen auf das Gewicht der Anwendungsmischung aus Mittel A, Mittel B und Mittel C, in dem anwendungsbereiten Färbemittel enthalten.

In diesem Zusammenhang kann der pH-Wert der finalen Anwendungsmischung, welche durch Vermischen der Mittel A, B und C hergestellt wird, zwischen 7,5 und 11 liegen. Liegt der pH-Wert der finalen Anwendungsmischung in einem Bereich von 8 bis 10, so ist dies bevorzugt.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierte Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurtehan und Polyesterfasern verwendet werden.

Das Mittel A enthält mindestens eine reaktive Carbonylverbindung.

Reaktive Carbonylverbindungen besitzen im Sinne der Erfindung mindestens eine Carbonylgruppe als reaktive Gruppe, welche mit der CH-aciden Komponente unter Ausbildung einer kovalenten Bindung reagiert. Bevorzugte reaktive Carbonylverbindungen sind ausgewählt aus Verbindungen die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente (Oxo2) stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente (Oxo1) leitet sich in diesem Fall c) von einem Aldehyd ab)
an das Kohlenstoffatom der Carbonylgruppe der reaktiven Carbonylverbindung.

Unter den reaktiven Carbonylverbindungen des Mittels A sind Verbindungen ausgewählt aus Verbindungen der folgenden Gruppe bevorzugt: Benzaldehyd und seine Derivate, Naphthaldehyd und seine Derivate, Zimtaldehyd und seine Derivate, 2-Formylmethylen-1,3,3-tri-methylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, Pyridoxal, Antipyrin-4-aldehyd, Furfural, 5-Nitrofurfural, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methyl-chinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat, -chlorid, -bromid, - iodid, -tetrachlorozinkat, -methylsulfat-, -trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Insbesondere bevorzugt ist es, wenn als reaktive Carbonylverbindung des Mittels A eine Verbindung ausgewählt aus mindestens einer Verbindungen der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 3-Carboxy-4-hydroxy-benzaldehyd, 5-Carboxyvanillin, 3-Carboxy-4-hydroxy-5-methylbenzaldehyd, 3-Carboxy-5-ethoxy-4-hydroxybenzaldehyd, 3-Carboxy-4-hydroxybenzaldehyd, 5-Carboxyvanillin, 3-Carboxy-4-hydroxy-5-methylbenzaldehyd, 3-Carboxy-5-ethoxy-4-hydroxybenzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-5-carboxy-4-hydroxybenzaldehyd (3-Allyl-5-formyl-2-hydroxybenzoesäure) und 3-Allyl-4-hydroxy-5-formylbenzaldehyd oder (5-Allyl-4-hydroxyisophthalaldehyd) eingesetzt wird.

Das Mittel B enthält mindestens eine CH-acide Verbindung der allgemeinen Formel (CH-1) und/oder der allgemeinen Formel (CH-2) *(vide supra*)*.*

Als CH-acide Verbindungen werden im Allgemeinen solche Verbindungen angesehen, die ein an ein aliphatischen Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt sind. Die Verbindungen gemäß Formel (CH-1) und (CH-2) sind C,H-acide Verbindungen. Aus den kationischen Verbindungen der Formel (CH-1) läßt sich durch Zugabe einer Base, welche die Abspaltung eines Protons bewirkt, gezielt die entsprechende ungeladene Enaminform darstellen. Exemplarisch für Verbindungen gemäß Formel (CH-1) wird die Darstellung der Enaminform nachfolgend anhand der Formeln (CH-1-A) und (CH-1-B) mit R⁷ = CH₃ illustriert. Auch die entsprechenden Enaminformen der CH-aciden Verbindungen gemäß Formel (CH-1) sind erfindungsgemäß.

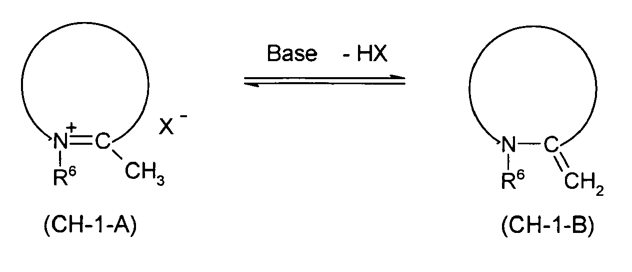

Es ist erfindungsgemäß besonders bevorzugt, die Verbindungen der Formel (CH-1) auszuwählen aus mindestens einer Verbindung der Formel (CH-3), 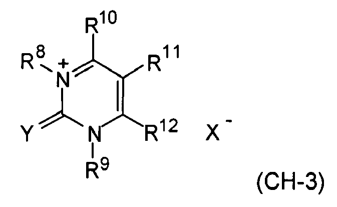 worin
- R⁸ und R⁹ stehen unabhängig voneinander für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R^{I}R^{II}N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₄)-alkylgruppe, wobei R' und R" gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine (C₁ bis C₆)-Alkylgruppe bedeutet,
- R¹¹ steht für ein Wasserstoffatom, eine (C₁ bis C₆)-Alkylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine (C₁ bis C₆)-Hydroxyalkoxygruppe, eine Gruppe R^{III}R^{IV}N-(CH₂)_{q}-, worin R^{III} und R^{IV} stehen unabhängig voneinander für ein Wasserstoffatom, eine (C₁ bis C₆)-Alkylgruppe, eine (C₁ bis C₆)-Hydroxyalkylgruppe oder eine Aryl-(C₁ bis C₆)-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer (C₁ bis C₆)-Alkylgruppe, einer (C₁ bis C₆)-Hydroxyalkylgruppe, einer (C₂ bis C₆)-P_{O}lyhydroxyalkylgruppe, einer (C₁ bis C₆)-Alkoxygruppe, einer (C₁ bis C₆)-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine (C₁ bis C₆)-Alkylgruppe, eine (C₁ bis C₆)-Hydroxyalkylgruppe oder eine Aryl-(C₁ bis C₆)-alkylgruppe und s steht für eine Zahl 0, 1, 2, 3, 4, 5 oder 6 substituiert sein kann,
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine (C₁ bis C₆)-Alkylgruppe oder eine Aryl-(C₁ bis C₆)-alkylgruppe,
- X- steht für ein physiologisch verträgliches Anion.

Mindestens eine Gruppe R¹⁰ oder R¹² gemäß Formel (CH-3) steht zwingend für eine (C₁ bis C₆)-Alkylgruppe. Diese Alkylgruppe trägt an deren α-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y der Formel (CH-3) für ein Sauerstoff- oder ein Schwefelatom, besonders bevorzugt für ein Sauerstoffatom.

Der Rest R⁸ der Formel (CH-3) wird bevorzugt ausgewählt aus einer (C₁ bis C₆)-Alkylgruppe (besonders bevorzugt einer Methylgruppe), einer (C₂ bis C₆)-Alkenylgruppe (insbesondere einer Allylgruppe), einer (C₂ bis C₆)-Hydroxyalkylgruppe (insbesondere eine 2-Hydroxyethylgruppe) oder einer gegebenenfalls substituierten Benzylgruppe.

R¹¹ der Formel (CH-3) steht bevorzugt für ein Wasserstoffatom.

Besonders bevorzugt stehen in Formel (CH-3) die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁ bis C₆)-Alkylgruppe (besonders bevorzugt einer Methylgruppe), einer (C₂ bis C₆)-Alkenylgruppe (insbesondere einer Allylgruppe), einer (C₂ bis C₆)-Hydroxyalkylgruppe (insbesondere eine 2-Hydroxyethylgruppe) oder einer gegebenenfalls substituierten Benzylgruppe.

Ganz besonders brillante Haarfärbungen können erhalten werden, wenn die in Mittel B enthaltene C,H-acide Verbindung der Formel (CH-1) und/oder der Formel (CH-2) eine Verbindung ausgewählt aus mindestens einer Verbindung der Gruppe bestehend aus 2-(2-Furoyl)-acetonitril, 2-(5-Brom-2-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1H-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 2-(6-Hydroxy-4,7-dimethoxy-1-benzofuran-5-oyl)-acetonitril und den Salzen mit physiologisch verträglichem Gegenion X⁻ des 1,2-Dihydro-1,3,4,6-tetramethyl-2-oxo-pyrimidiniums, 1,2-Dihydro-1,3-diethyl-4,6-dimethyl-2-oxo-pyrimidiniums, 1,2-Dihydro-1,3-dipropyl-4,6-dimethyl-2-oxo-pyrimidiniums, 1,2-Dihydro-1,3-di(2-hydroxyethyl)-4,6-dimethyl-2-oxo-pyrimidiniums, 1,2-Dihydro-1,3-diphenyl-4,6-dimethyl-2-oxo-pyrimidiniums, 1,2-Dihydro-1,3,4-trimethyl-2-oxo-pyrimidiniums, 1,2-Dihydro-1,3-diethyl-4-methyl-2-oxo-pyrimidiniums, 1,2-Dihydro-1,3-dipropyl-4-methyl-2-oxo-pyrimidiniums, 1,2-Dihydro-1,3-di(2-hydroxyethyl)-4-methyl-2-oxo-pyrimidiniums, 1,2-Dihydro-1,3-diphenyl-4-methyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums, 1,2-Dihydro-1,3,4,6-tetramethyl-2-thioxo-pyrimidiniums, 1,2-Dihydro-1,3-diethyl-4,6-dimethyl-2-thioxo-pyrimidiniums, 1,2-Dihydro-1,3-dipropyl-4,6-dimethyl-2-thioxo-pyrimidiniums, 1,2-Dihydro-1,3-di(2-hydroxyethyl)-4,6-dimethyl-2-thioxo-pyrimidiniums, 1,2-Dihydro-1,3-diphenyl-4,6-dimethyl-2-thioxo-pyrimidiniums, 1,2-Dihydro-1,3,4-trimethyl-2-thioxo-pyrimidiniums, 1 ,2-Dihydro-1,3-diethyl-4-methyl-2-thioxo-pyrimidiniums, 1,2-Dihydro-1,3-dipropyt-4-methyt-2-thioxo-pyrimidiniums, 1,2-Dihydro-1,3-di(2-hydroxyethyl)-4-methyl-2-thioxo-pyrimidiniums, 1,2-Dihydro-1,3-diphenyl-4-methyl-2-thioxo-pyrimidiniums, 1,2-Dihydro-3,4-dimethyl-2-oxo-chinazoliniums und 1,2-Dihydro-3,4-dimethyl-2-thioxo-chinazoliniums eingesetzt wird. Daher sind die Verbindungen dieser Gruppe besonders bevorzugt.

Die reaktiven Carbonylverbindungen des Mittels A werden vorzugsweise in einer Menge von 0,03 bis 65,00 mmol, bezogen auf 100 g des Mittels A, eingesetzt. Der Einsatz von 1,00 bis 30,00 mmol, bezogen auf 100 g des Mittels A, ist insbesondere bevorzugt.

Die CH-aciden Verbindungen der Formel (CH-1 ) und/oder der Formel (CH-2) in dem Mittel B werden vorzugsweise in einer Menge von 0,03 bis 65,00 mmol, bezogen auf 100 g des Mittels B, eingesetzt. Der Einsatz von 1,00 bis 30,00 mmol, bezogen auf 100 g des Mittels B, ist insbesondere bevorzugt.

In einer weiteren Ausführungsform kann in dem Mittel A und/oder in dem Mittel B und/oder in dem fakultativ einsetzbaren Mittel C zusätzlich mindestens eine Entwicklerkomponente und gegebenenfalls mindestens eine Kupplerkomponente als Oxidationsfarbstoffvorprodukte enthalten. Es ist jedoch bevorzugt die erfindungsgemäßen Mittel frei von Oxidationsfarbstoffvorprodukten zu formulieren, insbesondere wenn das Allergierisiko für para-Allergiker minimiert werden soll.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 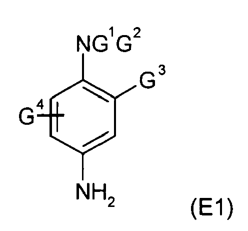 wobei
- G¹ steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen 4'-Aminophenylrest oder einen (C₁ bis C₄)-Alkylrest, der mit einer stickstoffhaltigen Gruppe, einem Phenyl- oder einem 4'-Aminophenylrest substituiert ist;
- G² steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest oder einen (C₁ bis C₄)-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴ steht für ein Wasserstoffatom, ein Halogenatom oder einen (C₁ bis C₄)-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 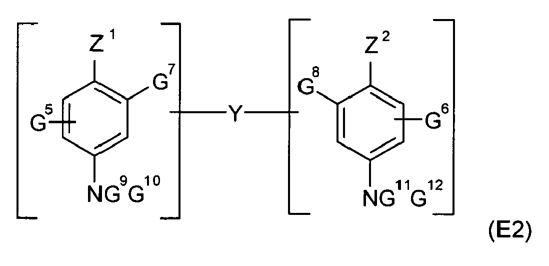 wobei:
- Z¹ und Z² stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶ stehen unabhängig voneinander für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)-phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 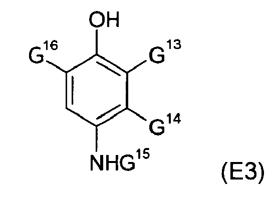 wobei:
- G¹³ steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴ steht für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵ steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methylp-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(a,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen, 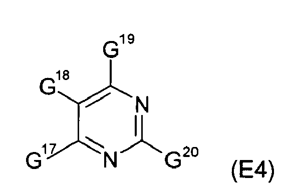 worin
- G¹⁷, G¹⁸ und G¹⁹ unabhängig voneinander für ein Wasserstoffatom, eine Hydroxygruppe, eine (C₁ bis C₄)-Alkoxygruppe oder eine Aminogruppe steht und
- G²⁰ für eine Hydroxygruppe oder eine Gruppe -NG²¹G²² steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷, G¹⁸, G¹⁹ und G²⁰ eine Hydroxygruppe bedeuten und höchstens zwei der Reste G¹⁷, G¹⁸ und G¹⁹ für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²² stehen und höchstens zwei Gruppen aus G¹⁷, G¹⁸, G¹⁹ und G²⁰ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 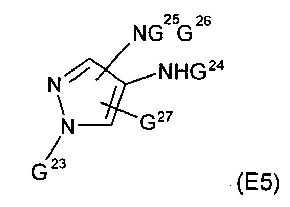 worin
- G²³, G²⁴, G²⁵ stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁶ neben den vorgenannten Gruppen zusätzlich für eine Gruppe -NH₂ stehen kann,
- G²⁶ steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷ steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²⁶ an die 5 Position und der Rest G²⁷ an die 3 Position des Pyrazolzyklus.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Bevorzugte Pyrazolopyrimidin-Derivate sind insbesondere die Derivate des Pyrazolo[1,5-a]pyrimidin der folgenden Formel (E6) und dessen tautomeren Formen, sofern ein tautomeres Gleichgewicht besteht: 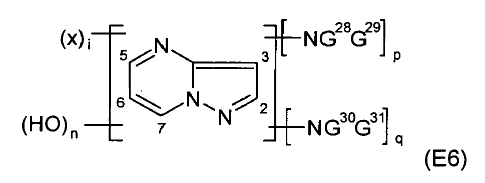 wobei:
- G²⁸, G²⁹ und G³⁰, G³¹ unabhängig voneinander stehen für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest einen (C₁ bis C₄)-Alkoxy-(C₁ bis C₄)-alkylrest, einen (C₁ bis C₄)-Aminoalkylrest, der gegebenenfalls durch ein Acetyl-Ureid- oder einen Sulfonyl-Rest geschützt sein kann, einen (C₁ bis C₄)-Alkylamino-(C₁ bis C₄)-alkylrest, einen Di-[(C₁ bis C₄)-alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Monohydroxyalkyl- oder einen Di[(C₁ bis C₄)-Hydroxyalkyl]-(C₁ bis C₄)-aminoalkylrest,
- die X-Reste stehen unabhängig voneinander für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest, einen (C₁ bis C₄)-Aminoalkylrest, einen (C₁ bis C₄)-Alkylamino-(C₁ bis C₄)-alkylrest, einen Di-[(C₁ bis C₄)alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Hydroxyalkyl- oder einen Di-[(C₁ bis C₄)-hydroxyalkyl]amino-(C₁ bis C₄)-alkylrest, einen Aminorest, einen (C₁ bis C₄)-Alkyl- oder Di-[(C₁ bis C₄)-hydroxyalkyl]aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG²⁸G²⁹ und NG³⁰G³¹ belegen die Positionen (2,3); (5,6); (6,7); (3,5) oder (3,7);
- wenn p + q gleich 1 ist, n den Wert 1 hat, und die Gruppen NG²⁸G²⁹ (oder NG³⁰G³¹) und die Gruppe OH belegen die Positionen (2,3); (5,6); (6,7); (3,5) oder (3,7);

Die in Formel (E7) verwendeten Substituenten sind erfindungsgemäß analog zu den obigen Ausführungen definiert.

Wenn das Pyrazolo[1,5-a]pyrimidin der obenstehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

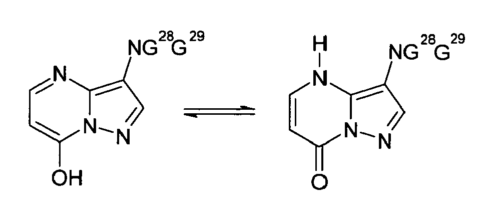

Unter den Pyrazolo[1,5-a]pyrimidinen der obenstehenden Formel (E7) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
   sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E6) können wie in der Literatur beschrieben durch Zyklisierung ausgehend von einem Aminopyrazol oder von Hydrazin hergestellt werden.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E6) genannten Reste aufgezählt: Beispiele für (C₁ bis C₄)-Alkylreste sind die Gruppen - CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, - OC(CH₃)₃, insbesondere eine Methoxy- oder eine Ethoxygruppe. Weiterhin können als bevorzugte Beispiele für eine (C₁ bis C₄)-Monohydroxyalkylgruppe - CH₂OH, -CH₂CH₂OH, -CH₂CH₂CH₂OH, -CHCH(OH)CH₃, -CH₂CH₂CH₂CH₂OH, wobei die Gruppe - CH₂CH₂OH bevorzugt ist.

Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylamino-gruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺. Beispiele für (C₁ bis C₄)-Monoalkylaminogruppen sind -NHCH₃, -NHCH₂CH₃, - NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Trialkylammoniumgruppen sind -N⁺(CH₃)₃, -N⁺(CH₃)₂(CH₂CH₃), -N⁺(CH₃)(CH₂CH₃)₂.
Beispiele für (C₁ bis C₄)-Hydroxyalkylaminoreste sind -NH-CH₂CH₂OH, -NH-CH₂CH₂OH, -NH-CH₂CH₂CH₂OH, -NH-CH₂CH₂CH₂OH.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).
Beispiele für Hydroxy-(C₁ bis C₄)-alkoxyreste sind -O-CH₂OH, -O-CH₂CH₂OH, -O-CH₂CH₂CH₂OH, -O-CHCH(OH)CH₃, -O-CH₂CH₂CH₂CH₂OH.
Beispiele für (C₁ bis C₄)-Acetylaminoalkoxyreste sind -O-CH₂NHC(O)CH₃, -O-CH₂CH₂NHC(O)CH₃, -O-CH₂CH₂CH₂NHC(O)CH₃, -O-CH₂CH(NHC(O)CH₃)CH₃, -O-CH₂CH₂CH₂CH₂NHC(O)CH₃.
Beispiele für (C₁ bis C₄)-Carbamoylaminoalkoxyreste sind -O-CH₂CH₂-NH-C(O)-NH₂, -O-CH₂CH₂CH₂-NH-C(O)-NH₂, -O-CH₂CH₂CH₂CH₂NH-C(O)-NH₂.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Beispiele für (C₁ bis C₄)-Cyanoalkylreste sind -CH₂CN, -CH₂CH₂CN, -CH₂CH₂CH₂CN.
Beispiele für (C₁ bis C₄)-Hydroxyalkylamino-(C₁ bis C₄)-alkylreste sind -CH₂CH₂NH-CH₂CH₂OH,

### -CH₂CH₂CH₂NH-CH₂CH₂OH, -CH₂CH₂NH-CH₂CH₂CH₂OH, -CH₂CH₂CH₂NH-CH₂CH₂CH₂OH.

Beispiele für Di[(C₁ bis C₄)-Hydroxyalkyl]amino-(C₁ bis C₄)-alkylreste sind - CH₂CH₂N(CH₂CH₂OH)₂,

### -CH₂CH₂CH₂N(CH₂CH_{Z}OH)₂, -CH₂CH₂N(CH₂CH₂CH_{Z}OH)₂, -CH₂CH₂CH₂N (CH₂CH₂CH₂OH)₂.

Ein Beispiel für Arylgruppen ist die Phenylgruppe.
Beispiele für Aryl-(C₁ bis C₄)-alkylgruppen sind die Benzylgruppe und die 2-Phenylethylgruppe.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Enfinricklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Aminobenzomorpholin,
- Chinoxalinderivate wie beispielsweise 6-Methyl-1,2,3,4-tetrahydrochinoxalin,
   Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1), 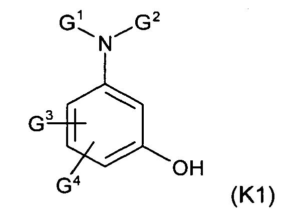 worin
- G¹ und G²: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₂ bis C₄)-Perfluoracylgruppe, eine Aryl-(C₁ bis C₆)-alkylgruppe, eine Amino-(C₁ bis C₆)-alkylgruppe, eine (C₁ bis C₆)-Dialkylamino-(C₁ bis C₆)-alkylgruppe oder eine (C₁ bis C₆)-Alkoxy-(C₁ bis C₆)-alkylgruppe, wobei G¹ und G² gemeinsam mit dem Stickstoffatom einen fünfgliedrigen, sechsgliedrigen oder siebengliedrigen Ring bilden können,
- G³ und G⁴: unabhängig voneinander stehen für ein Wasserstoffatom, ein Halogenatom, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe, eine (C₁ bis C₆)-Alkyox-(C₂ bis C₆)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 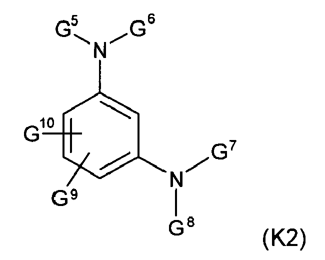 worin
- G⁵, G⁶, G⁷ und G⁸: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G⁹ und G¹⁰: unabhängig voneinander stehen für ein Wasserstoffatom, ein Halogenatom, eine (C₁ bis C₄)-Alkylgruppe, eine w-(2,4-Diaminophenyl)-(C₁ bis C₄)-alkylgruppe, eine ω-(2,4-Diaminophenyloxy)-(C₁ bis C₄)-alkoxygruppe, eine (C₁ bis C₄)-Alkoxygruppe, eine Hydroxygruppe, eine (C₁ bis C₄)-Alkoxy-(C₂ bis C₄)-alkoxygruppe, eine Arylgruppe, eine Heteroarylgruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 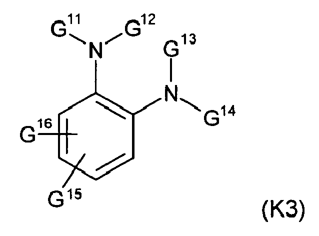 worin
- G¹¹, G¹², G¹³ und G¹⁴: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G¹⁵ und G¹⁶: unabhängig voneinander stehen für ein Wasserstoffatom, ein Halogenatom, eine Carboxylgruppe, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 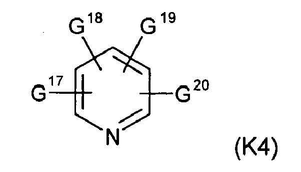 worin
- G¹⁷ und G¹⁸: stehen unabhängig voneinander für eine Hydroxygruppe oder eine Gruppe-NG²¹G²² , worin G²¹ und G²² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine Arylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe,
- G¹⁹ und G²⁰: stehen unabhängig voneinander für ein Wasserstoffatom, ein Halogenatom, eine (C₁ bis C₄)-Alkylgruppe oder eine (C₁ bis C₄)-Alkoxygruppe.

Es ist bevorzugt, wenn gemäß Formel (K4) die Reste G¹⁷ und G¹⁸ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 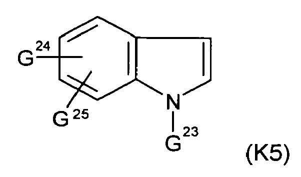 worin
- G²³: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁴: steht für eine Hydroxygruppe oder eine Gruppe -NG²⁶G²⁷, worin G²⁶ und G²⁷ unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G²⁵: Wasserstoffatom, ein Halogenatom oder eine (C₁ bis C₄)-Alkylgruppe,
mit der Maßgabe, dass G²⁴ in meta-Position oder ortho-Position zum Strukturfragment NG²³ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 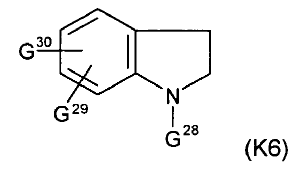 worin
- G²⁸: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁹: steht für eine Hydroxygruppe oder eine Gruppe -NG³¹G³², worin G³¹ und G³² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G³⁰: Wasserstoffatom, ein Halogenatom oder eine (C₁ bis C₄)-Alkylgruppe,
mit der Maßgabe, dass G²⁹ in meta-Position oder ortho-Position zum Strukturfragment NG²⁸ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Gemisch aus Mittel A, Mittel B und gegebenenfalls Mittel C, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für (C₁ bis C₄)-Alkylreste sind die Gruppen - CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.
Erfindungsgemäße Beispiele für (C₃ bis C₆)-Cycloalkylgruppen sind die Cyclopropyl, die Cyclopentyl und die Cyclohexylgruppe.
Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, - OC(CH₃)₃, insbesondere eine Methoxy- oder eine Ethoxygruppe.
Weiterhin können als bevorzugte Beispiele für eine (C₁ bis C₄)-Monohydroxyalkylgruppe - CH₂OH -CH₂CH₂OH, -CH₂CH₂CH₂OH -CH₂CH(OH)CH₃, -CH₂CH₂CH₂CH₂OH genannt werden, wobei die Gruppe -CH₂CH₂OH bevorzugt ist.
Ein besonders bevorzugtes Beispiel einer (C₂ bis C₄)-Polyhydroxyalkylgruppe ist die 1,2-Dihydroxyethylgruppe.

Beispiele für Halogenatome sind F-, CI- oder Br-Atome, CI-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.
Beispiele für (C₁ bis C₄)-Monoalkylaminogruppen sind -NHCH₃, -NHCH₂CH₃, - NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃)₂, -CH₂CH₂CH₂-O-CH(CH₃)₂.
Beispiele für (C₁ bis C₄)-Alkoxy-(C₁ bis C₄)-alkoxygruppen sind die Gruppen -O-CH₂CH₂-O-CH₃, -O-CH₂CH₂CH₂-O-CH₃, -O-CH₂CH₂-O-CH₂CH₃, -O-CH₂CH₂CH₂-O-CH₂CH₃, -O-CH₂CH₂-O-CH(CH₃)₂, -O-CH₂CH₂CH₂-O-CH(CH₃)₂.
Beispiele für Hydroxy-(C₁ bis C₄)-alkoxyreste sind -O-CH₂OH, -O-CH₂CH₂OH, -O-CH₂CH₂CH₂OH,
-O-CH₂CH(OH)CH₃, -O-CH₂CH₂CH₂CH₂OH.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Ein Beispiel für Arylgruppen ist die Phenylgruppe, die auch substituiert sein kann.
Beispiele für Aryl-(C₁ bis C₄)-alkylgruppen sind die Benzylgruppe und die 2-Phenylethylgruppe.

Es kann erfindungsgemäß bevorzugt sein, den Mitteln A und/oder B des erfindungsgemäßen Kits und/oder dem gegebenenfalls zusätzlich enthaltenen Mittel C als weitere Farbstoffvorstufe einen naturanalogen Farbstoff zuzusetzen.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins der Formel (RN1), 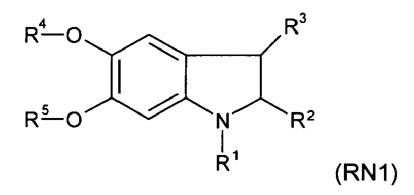 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (RN2), 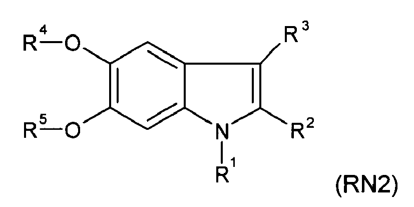 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- beziehungsweise die Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Weiterhin kann in dem Mittel A und/oder in dem Mittel B und/oder in dem fakultativ einsetzbaren Mittel C zusätzlich mindestens ein direktziehender Farbstoff enthalten sein. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.%.

Direktziehende Farbstoffe können in anionische, kationische und nichtionische direktziehende Farbstoffe unterteilt werden.

### Anionische direktziehende Farbstoffe:

Als anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono-und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobezolsulfonsäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure-natriumsalz (C.I. 10,385; Acid Orange 3), 4-[(2,4- Dihydroxyphenyl)azo]-benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]-benzolsulfonsäure-natriumsalz (C.I. 20,170; Acid Orange 24), 4-Hydroxy-3-[(2-methoxyphenyl)azo]-1-naphthalinsulfonsäure-natriumsalz (C.I. 14,710; Acid Red 4), 4-Hydroxy-3-[(4-sulfonaphth-1-yl)azo]-1-naphthalin-sulfonsäure-dinatriumsalz (C.I. 14,720; Acid Red No.14), 6- Hydroxy-5-[(4-sulfonaphth-1-yl)azo]-2,4-naphthalin-disulfonsäure-trinatriumsalz (C.I. 16,255; Ponceau 4R; Acid Red 18), 3-Hydroxy-4-[(4-sulfonaphth-1-yl)azo]-2,7-naphthalin-disulfonsäure-trinatriumsalz (C.I. 16,185; Acid Red 27), 8-Amino-1-hydroxy-2-(phenylazo)-3,6-naphthalin-disulfonsäure-dinatriumsalz (C.I. 17,200; Acid Red 33; Red 33), 5- (Acetylamino)-4-hydroxy-3-[(2-methylphenyl)azo]-2,7-naphthalin-disulfonsäure-dinatriumsalz (C.I. 18,065; Acid Red 35), 2-(3-Hydroxy-2,4,5,7-tetraiod-dibenzopyran-6-on-9-yl)-benzoesäure-dinatriumsalz (C.I. 45,430; Acid Red 51), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 8-[(4-(Phenylazo)phenyl)azo]-7-naphthol-1,3-disulfonsäure-dinatriumsalz (C.I. 27,290; Acid Red 73), 2',4',5',7'-Tetrabrom-3',6'-dihydroxyspiro[isobenzofuran-1(3H),9'-[9H]xanthen]-3-on-dinatriumsalz (C.I. 45,380; Acid Red 87), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3',6'-Dihydroxy-4',5'-diiodospiro[isobenzofuran-1(3H),9'(9H)- xanthen]-3-on-dinatriumsalz (C.I. 45425; Acid Red 95), 2-Hydroxy-3-((2- hydroxynaphth-1-yl)azo)-5-nitrobenzolsulfonsäure-natriumsalz (C.I. 15,685; Acid Red 184), 3-Hydroxy-4-(3-methyl-5-oxo-1-phenyl-4,5-dihydro-1H-pyrazol-4-ylazo)-naphthalin-1-sulfonsäure-natriumsalz, Chrom-Komplex (Acid Red 195), 3-Hydroxy-4-[(4-methyl-2-sulfonphenyl)azo]-2-naphthalincarbonsäure-calciumsalz (C.I. 15,850:1; Pigment Red 57:1), 3-[(2,4-Dimethyl-5-sulfophenyl)azo]-4-hydroxy-1-naphthalin-sulfonsäure-dinatriumsalz (C.I. 14,700; Food Red No. 1; Ponceau SX; FD&C Red No. 4), 1,4- Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)-phenyl](2,4-disulfophenyl)carbenium-inneres Salz, Natriumsalz (2:1) (C.I. 42,045; Food Blue No. 3; Acid Blue 1), Bis[4-(diethylamino)phenyl](5-hydroxy-2,4-disulfophenyl)-carbenium-inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure-natriumsalz (C.I. 62045; Acid Blue 62), 2-(1,3-Dihydro-3-oxo-5-sulfo-2H-indol-2-yliden)-2,3-dihydro-3-oxo-1 H-indol-5- sulfonsäure-dinatriumsalz (C.I. 73,015; Acid Blue 74), 9-(2-Carboxyphenyl)-3-[(2-methylphenyl)amino]-6-[(2-methyl-4-sulfophenyl)amino]xanthylium-inneres Salz, Natriumsalz (C.I. 45,190; Acid Violet 9), 1-Hydroxy-4-[(4-methyl-2- sulfophenyl)amino]-9,10-anthrachinon-natriumsalz (C.I. 60,730; D&C Violett No. 2; Acid Violet 43), Bis[3-nitro-4-[(4-phenylamino)-3-sulfo-phenylamino]-phenyl]-sulfon (C.I. 10,410; Acid Brown 13), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure-tetranatriumsalz (C.I. 28,440; Food Black No. 1), 3',3",5',5"-Tetrabromphenolsulfonphthalein (Bromphenolblau).

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

### Kationische direktziehende Farbstoffe:

Als kationische direktziehende Farbstoffe eignen sich insbesondere 9-(Dimethylamino)-benzo[a]phenoxazin-7-ium-chlorid (C.I. 51,175; Basic Blue 6), Di[4-(diethylamino)phenyl][4-(ethylamino)naphthyl]carbenium-chlorid (C.I. 42,595; Basic Blue 7), Di-(4-(dimethylamino)-phenyl)-(4-(methyl-phenylamino)-naphthalin-1-yl)carbenium-chlorid (C.I. 42,563; Basic Blue 8), 3,7-Di(dimethylamino)phenothiazin-5-ium-chlorid (C.I. 52,015 Basic Blue 9), Di[4-(di-methylamino)phenyl][4-(phenylamino)naphthyl] carbenium-chlorid (C.I. 44,045; Basic Blue 26), 2-[(4-(Ethyl(2-hydroxyethyl)amino)phenyl)azo]-6-methoxy-3-methyl-benzothiazolium-methylsulfat (C.I. 11,154; Basic Blue 41), 8-Amino-2-brom-5-hydroxy-4-imino-6-[(3-(trimethylammonio)phenyl)amino]-1 (4H)-naphthalinon-chlorid (C.I. 56,059; Basic Blue No. 99), Bis[4- (dimethylamino)phenyl]-[4-(methylamino)phenyl]carbenium-chlorid (C.I. 42,535; Basic Violet 1), Tri(4-amino-3-methylphenyl)carbenium-chlorid (C.I. 42,520; Basic Violet 2), Tri[4-(dimethylamino)-phenyl]carbenium-chlorid (C.I. 42,555; Basic Violet 3), 2-[3,6-(Diethylamino)dibenzopyranium-9-yl]- benzoesäurechlorid (C.I. 45,170; Basic Violet 10), Di(4-aminophenyl)(4-amino-3- methylphenyl)carbeniumchlorid (C.I. 42,510 Basic Violet 14), 1,3-Bis[(2,4-diamino-5-methylphenyl)azo]-3-methylbenzol (C.I. 21,010; Basic Brown 4), 1-[(4-Aminophenyl)azo]-7-(trimethylammonio)- 2-naphthol-chlorid (C.I. 12,250; Basic Brown 16), 1-[(4-Amino-2-nitrophenyl)azo]-7-(trimethylammonio)-2-naphtholchlorid, 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminiumchlorid (C.I. 12,605, Basic Orange 69), 3,7-Diamino-2,8-dimethyl- 5-phenylphenazinium-chlorid (C.I. 50,240; Basic Red 2), 1,4-Dimethyl-5-[(4-(dimethylamino)phenyl)azo]-1,2,4-triazolium-chlorid (C.l. 11,055; Basic Red 22), 2-Hydroxy-1-[(2-methoxyphenyl)azo]-7-(trimethylammonio)-naphthalin-chlorid (C.I. 12,245; Basic Red 76), Di[4-(dimethylamino)phenyl]iminomethan-hydrochlorid (C.I. 41,000; Basic Yellow 2), 2-[2-((2,4- Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]-pyrazol-5-on-chlorid (C.I. 12,719; Basic Yellow 57), Bis[4-(diethylamino)phenyl]phenylcarbenium-hydrogensulfat (1:1) (C.I. 42,040; Basic Green 1), Di(4- (dimethylamino)phenyl)-phenylmethanol (C.I. 42,000; Basic Green 4), 1-(2-Morpholiniumpropylamino)-4-hydroxy-9,10-anthrachinon-methylsulfat, 1-[(3-(Dimethyl-propylaminium)-propyl)amino]-4-(methylamino)-9,10-anthrachinon-chlorid und direktziehende Farbstoffe, die einen Heterocyclus enthalten, der mindestens ein quaternäres Stickstoffatom aufweist.

Bevorzugte kationische direktziehenden Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

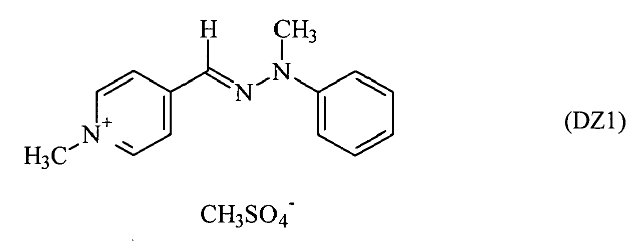

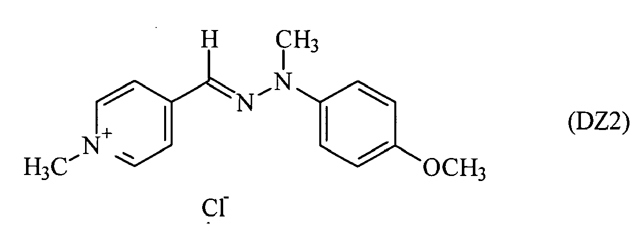

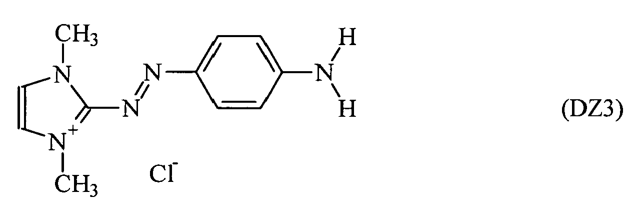

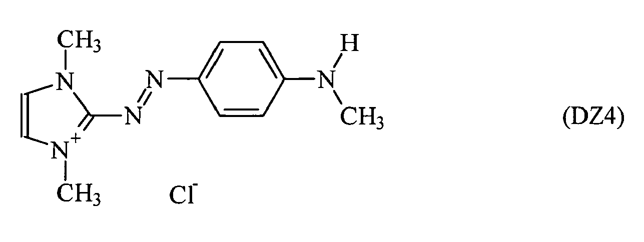

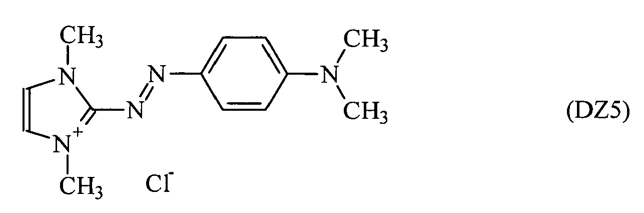

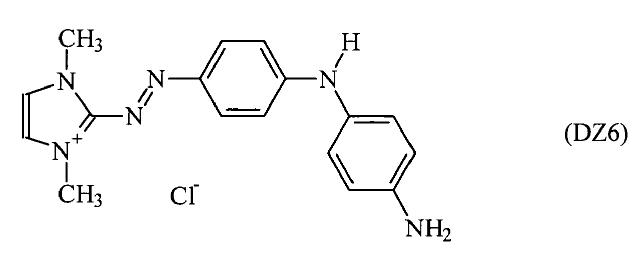

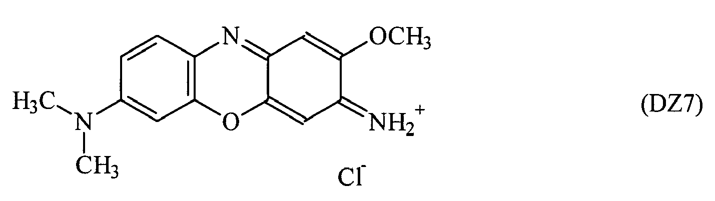

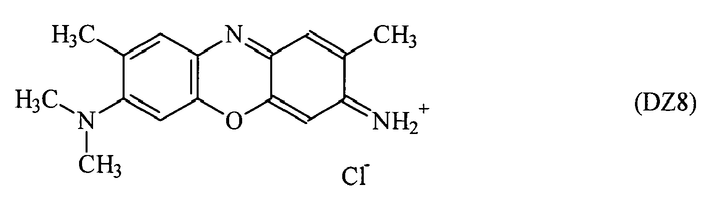

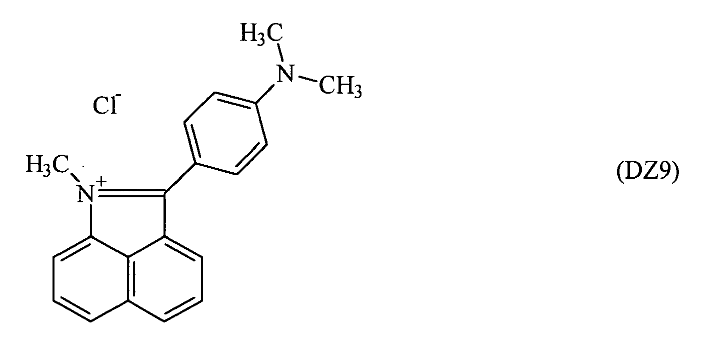

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

### Nichtionische direktziehende Farbstoffe:

Als nichtionische direktziehende Farbstoffe eignen sich insbesondere nichtionische Nitro-und Chinonfarbstoffe und neutrale Azofarbstoffe.

Geeignete blaue Nitrofarbstoffe sind insbesondere:
1,4-Bis[(2-hydroxyethyl)amino]-2-nitrobenzol, 1-(2-Hydroxyethyl)amino-2-nitro-4-[di(2-hydroxyethyl)amino]-benzol (HC Blue 2), 1-Methylamino-4- [methyl-(2,3-dihydroxypropyl)-amino]-2-nitrobenzol (HC Blue 6),1-[(2,3- Dihydroxypropyl)-amino]-4-[ethyl-(2-hydroxyethyl)-amino]-2-nitrobenzol-hydrochlorid (HC Blue 9), 1-[(2,3-Dihydroxypropyl)amino]-4-[methyl-(2= hydroxyethyl)amino]-2-nitrobenzol (HC Blue 10), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2), 1-(2-Aminoethylamino)-4-[di(2-hydroxyethyl)amino]-2-nitrobenzol, 4-(Di(2-hydroxyethyl)amino)-2-nitro-1-phenylamino-benzol.

Geeignete rote Nitrofarbstoffe sind insbesondere:
1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methyl-amino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)-amino]-benzol, 4-Amino-3-nitrophenol, 4-[(2-Hydroxyethyl)-amino]-3-nitrophenol, 4-((2-Nitrophenyl)amino]-phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3- Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14).

Geeignete gelbe Nitrofarbstoffe sind insbesondere:
1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[Di(2-hydroxyethyl)amino]-5-nitrophenol, 2-[(2-Hydroxyethyl)amino]-1 -methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13), 4-[(2-Hydroxyethyl)-amino]-3-nitro-benzonitril (HC Yellow 14), 4-[(2-Hydroxyethyl)amino]-3-nitro-benzamid (HC Yellow 15) 3-[(2-Hydroxyethyl)-amino]-4-methyl-1 -nitrobenzol, 4-Chlor-3-[(2-hydroxyethylamino]-1-nitrobenzol.

Geeignete Chinonfarbstoffe sind insbesondere:
1,4-Di[(2,3-dihydroxypropyl)amino]-9,10-anthrachinon, 1,4-Di[(2-hydroxyethyl)amino]-9,10-anthrachinon (C.I. 61,545, Disperse Blue 23), 1-[(2-Hydroxyethyl)amino]-4-methylamino-9,10-anthrachinon (C.1. 61,505, Disperse Blue 3), 2-[(2-Aminoethyl)amino]-9,10-anthrachinon (HC Orange 5), 1-Amino-4-hydroxy-9,10-anthrachinon (C.I. 60,710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbon-säure (C.I. 75,470, Natural Red 4) , 1-[(3-Aminopropyl)amino]-4-methylamino-9,10-anthrachinon (HC Blue 8), 1-[(3-Aminopropyl)-amino]-9,10- anthrachinon (HC Red 8), 1,4-Diamino-2-methoxy-9,10-anthrachinon (C.I. 62,015, Disperse Red 11, Solvent Violet No. 26), 1,4-Dihydroxy-5,8-bis[(2-hydroxyethyl)amino]-9,10-anthrachinon (C.I. 62,500, Disperse Blue 7, Solvent Blue No. 69), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (C.I. 75,500, Natural Brown 7), 2-Hydroxy-1,4-naphthochinon (C.I. 75,480, Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl) amino]-1-methyl-1H- pyrazol-4-yl}imino}-4,5-dihydro-5-[(2-hydroxyethyl)-imino]-1-methyl-1H-Pyrazol-sulfat(1:1), Hydrat(1:1).

Geeignete neutrale Azofarbstoffe sind insbesondere:
1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4-Aminophenyl)azo]-1-[di(2-hydroxyethyl)amino]-3-methylbenzol (HC Yellow 7), 2,6-Diamino-3-[(pyridin-3-yl)azo]-pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel können die fakultativ enthaltenen Entwickler, Kuppler und/oder Direktzieher in einer Menge von 0,01 bis 20,00 Gew.-%, bezogen auf den erfindungsgemäßen Kit und damit bezogen auf eine Mischung aus Mittel A und Mittel B und gegebenenfalls zusätzlich Mittel C enthalten.

Weiterhin kann der erfindungsgemäße Kit auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Zur Erlangung weiterer und intensiverer Ausfärbungen können Mittel A und/oder Mittel B und/oder das darüber hinaus fakultativ einsetzbare Mittel C zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g der fertigen Anwendungsmischung, eingesetzt werden.

Auf die Anwesenheit von Oxidationsmitteln, z. B. H₂O₂, kann, insbesondere wenn der erfindungsgemäße Kit keine Oxidationsfarbstoffvorprodukte enthält, verzichtet werden. Wenn der erfindungsgemäße Kit luftoxidable Oxidationsfarbstoffvorprodukte oder Indol bzw. Indolinderivate enthält, kann in einem solchen Fall ohne Probleme auf Oxidationsmittel verzichtet werden. Es kann jedoch u. U. wünschenswert sein, dem erfindungsgemäßen Kit zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die fertige Anwendungmischung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Weiterhin kann der erfindungsgemäße Kit alle in solchen Zubereitungen bekannten Wirk-, Zusatz- und Hilfsstoffe enthalten. In vielen Fällen enthalten die anwendungsbereiten Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich im erfindungsgemäßen Kit alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammonium-glycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl-oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in dem erfindungsgemäßen Kit verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacry-lat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des kosmetischen Trägers werden zur Herstellung des Mittels A, des Mittels B und gegebenenfalls zusätzlich des Mittels C in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% bezogen auf das jeweilige Mittel eingesetzt.

Ein zweiter Gegenstand der vorliegenden Erfindung betrifft die Verwendung eines kosmetischen Trägermediums mit einem pH-Wert von 2 bis 5 zur Stabilisierung von darin enthaltenen reaktiven Carbonylverbindungen.

Als reaktive Carbonylverbindungen können insbesondere deren vorstehend benannte bevorzugte und besonders bevorzugte Vertreter eingesetzt werden.

Ein dritter Gegenstand der vorliegenden Erfindung betrifft die Verwendung eines kosmetischen Trägermediums mit einem pH-Wert von 0,5 bis 2,5 zur Stabilisierung von darin enthaltenen C,H-aciden Verbindungen.

Als C,H-acide Verbindung ist bevorzugt mindestens ein Vertreter ausgewählt aus den bereits beschriebenen Substanzen der Formel (CH-1) und/oder (CH-2) einzusetzen.

Ein vierter Gegenstand der vorliegenden Erfindung betrifft ein Verfahren zum Färben von keratinhaltigen Fasern, insbesondere menschlichen Haaren, dadurch gekennzeichnet dass
- mindestens ein Mittel A, das in einem kosmetischen Träger mindestens eine reaktive Carbonylverbindung enthält und einen pH-Wert von 2 bis 5 aufweist und
- mindestens ein Mittel B, das in einem kosmetischen Träger mindestens eine CH-acide Verbindung ausgewählt aus Verbindungen der Formel (CH-1 ) und/oder Verbindungen der Formel (CH-2) enthält 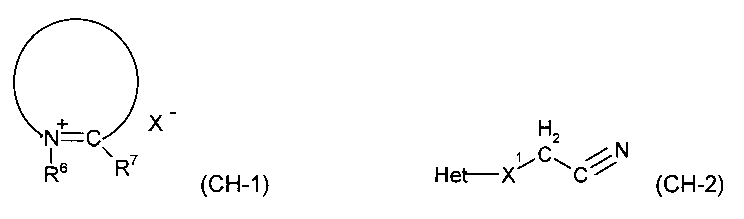 worin
   - R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe R^{I}R^{II}N-(CH₂)ₘ-, worin R' und R" stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R' und R" gemeinsam mit dem Stickstoffatom einen 5-, 6- oder 7-gliedrigen Ring bilden können und m steht für eine Zahl 2, 3, 4, 5 oder 6,
   - R⁷ steht für eine (C₁ bis C₆)-Alkylgruppe, insbesondere für eine Methylgruppe,
   - X- steht für ein physiologisch verträgliches Anion,
   - der Zyklus der Formel (CH-1 ) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
   - Het steht für einen gegebenenfalls substituierten Heteroaromaten,
   - X¹ steht für eine direkte Bindung oder eine Carbonylgruppe
      und einem pH-Wert von 0,5 bis 2,5 aufweist,
miteinander vermischt werden, die resultierende Mischung auf die keratinhaltige Faser aufgebracht, 5 bis 60 Minuten auf der Faser belassen und anschießend wieder ausgespült oder mit einem Shampoo ausgewaschen wird.

In diesem Zusammenhang ist es bevorzugt, die fertige, nach dem Zusammenmischen der Mittel A und B resultierende Anwendungsmischung für einen Zeitraum von 15 bis 45 Minuten auf dem Haar zu belassen.

In einer besonders bevorzugten Ausführungsform dieses Verfahrens kann die Mischung aus Mittel A und Mittel B durch Zugabe mindestens eines weiteren Mittels C auf einen alkalischen pH-Wert gebracht werden, wobei das Mittel C in einem kosmetischen Träger mindestens ein Alkalisierungsmittel enthält und einen pH-Wert größer als 7 aufweist.

In diesem Fall wird das Verfahren dahingehend modifiziert, dass die Komponenten A, B und C vor dem Aufbringen auf die Haarfaser innig miteinander vermischt werden. Diese Mischung wird dann auf das Haar aufgebracht und dort 5 bis 60 Minuten, bevorzugt 15 bis 45 Minuten, belassen.

Hierbei ist die Reihenfolge des Vermischens der Mittel A, B und C unerheblich. So kann erst Mittel A mit Mittel B vermengt werden, wobei die hierbei resultierende Mischung anschließend mit Mittel C versetzt und weiter durchmischt wird. Genauso ist es aber denkbar, erst Mittel A mit Mittel C zu vermischen und Mittel B nachträglich zuzusetzen bzw. Mittel B mit Mittel C zu vermischen und Mittel A nachträglich zuzusetzen.

Zudem ist es denkbar, dass die Mischung der Mittel A und B erst auf der keratinhaltigen Faser erfolgt. In diesem Fall wird zuerst nur das Mittel A vom Verbraucher appliziert und gegebenenfalls 5 bis 15 Minuten einwirken gelassen, bevor durch die nachfolgende Anwendung des Mittels B die Vermengung erfolgt. Die umgekehrte Anwendungsreihenfolge in Form der zuerst ausgeführten Anwendung des Mittels B mit nachfolgender Applikation des Mittels A ist ebenfalls möglich. Das Mittel C kann, wenn es zur Anwendung kommt, in diesem Zusammenhang vor dem Auftragen auf die Haarfaser mit einem der Mittel A oder B vermischt werden oder aber ebenfalls getrennt auf das Haar appliziert werden.

Wenn bei dem erfindungsgemäßen Verfahren zusätzlich ein Mittel C eingesetzt wird, ist es bevorzugt, wenn der pH-Wert der resultierenden Mischung aus Mittel A, Mittel B und Mittel C im alkalischen Bereich bei einem Wert größer 7 liegt. Ein pH-Wert von 7,5 bis 11 ist bevorzugt. Liegt der pH-Wert der finalen Anwendungsmischung zwischen 8 und 10,5 so ist dies besonders bevorzugt.

Während der Einwirkzeit der erfindungsgemäßen Kits auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partei mit einer Haube abgedeckt.

Nach dem Färbevorgang wird der erfindungsgemäße Kit vom Haar wieder abgespült oder mit einem Shampoo ausgewaschen. Als Nachbehandlung kann zusätzlich eine Haarkur oder ein Conditioner angewendet werden.

### Beispiele

### 1.1. Herstellung von Formulierungen des Mittels A

Für das Mittel A wurden aus den in Tabelle 1 bis Tabelle 5 aufgeführten Rezepturbestandteilen die nachfolgenden Formulierungen wie folgt hergestellt:

Zunächst wurden Lorol, Eumulgin B1 bzw. Eumulgin B2 und/oder Hydrenol D zusammen bei 80 °C aufgeschmolzen. In diese Schmelze wurden dann bei 80 °C die für die Einzelformulierungen notwendigen Mengenanteile der Komponenten Texapon NSO, Plantacare 1200, Plantacare 2000, Dehyton K, Dehyton AB 30, Crodaphos CES und/oder Crodaphos MCA unter Rühren eingearbeitet. Dieses Gemisch wurde langsam unter Rühren auf Raumtemperatur abkühlen gelassen. Während dieses Abkühlvorganges wurden die übrigen Rezepturbestandteile Incroquat DCMC, Incroquat Behenyl TMS, Hydrotriticum QM, Abil B 9905, Merquat 280, Methylparaben, Propylparaben, Phenoxyethanol, Na-Salicylat, Wasserglas, Imidazol, Turpinal SL und/oder Parfum hinzugefügt. Anschließend wurden die reaktiven Carbonylverbindungen 4-Hydroxy-2-methoxybenzaldehyd, 4-Hydroxy-3-methoxybenzaldehyd, 3,5-Dimethoxy-4-hydroxybenzaldehyd, 4-Hydroxy-1-naphthaldehyd und/oder 4-Formyl-1-methyl-chinolinium-p-toluolsulfonat sowie gegebenenfalls der Direktzieher HC Yellow 2 eingearbeitet. Dieses sorgfältig vermengte Gemisch wurde mit 80 bis 90 °C heißem Wasser auf 100 g aufgefüllt und erneut unter Rühren abkühlen gelassen. Zum Schluß wurde durch Zugabe von Weinsäure bzw. Monoethanolamin der für die jeweilige Formulierung angegebene pH-Wert eingestellt. Der pH-Wert wurde bei 22 °C gemessen.

Die dem Stand der Technik entsprechenden Vergleichsformulierungen sind mit VA1 bis VA10 beziffert. Bei diesen Formulierungen wurde durch Zugabe von Monoethanolamin ein alkalischer pH-Wert eingestellt. Die erfindungsgemäßen Formulierungen A1 bis A10 wurden durch Zugabe von Weinsäure auf einen sauren pH-Wert eingestellt.

**Tabelle 1**

| **Komponente** | **A1** | **VA1** | **A2** | **VA2** |
|---|---|---|---|---|
| 4-Hydroxy-2-methoxybenzaldehyd | 5 mmol | 5 mmol | 5 mmol | 5 mmol |
| 4-Hydroxy-3-methoxybenzaldehyd | - | - | - | - |
| 3,5-Dimethoxy-4-hydroxybenzaldehyd | 5 mmol | 5 mmol | - | - |
| 4-Hydroxy-1-naphthaldehyd | - | - | - | - |
| 4-Formyl-1-methyl-chinolinium-p- toluolsulfonat | - | - | 5 mmol | 5 mmol |
| HC Yellow 2 | 5 mmol | 5 mmol | 5 mmol | 5 mmol |
| Lorol, techn. | 2,5 g | 2,5 g | 2 g | 2 g |
| Eumulgin B2 | 0,8 g | 0,8 g | 1 g | 1 g |
| Eumulgin B1 | - | - | 0,4 g | 0,4 g |
| Hydrenol D | 8,5 g | 8,5 g | 8 g | 8 g |
| Texapon NSO | 15 g | 15 g | - | - |
| Plantacare 1200 | - | - | - | - |
| Plantacare 2000 | - | - | - | - |
| Dehyton K | 11,5 g | 11,5 g | 11,5 g | 11,5 g |
| Dehyton AB 30 | - | - | - | - |
| Crodaphos CES | - | - | 5 g | 5 g |
| Crodaphos MCA | - | - | - | - |
| Incroquat DCMC | 2 g | 2 g | - | - |
| Incroquat Behenyl TMS | - | - | 3 g | 3 g |
| Hydrotriticum QM | - | - | - | - |
| Abil B 9905 | - | - | - | - |
| Merquat 280 | - | - | - | - |
| Methylparaben | 0,2 g | 0,2 g | 0,3 g | 0,3 g |
| Propylparaben | 0,2 g | 0,2 g | 0,2 g | 0,2 g |
| Phenoxyethanol | 0,5 g | 0,5 g | 0,7 g | 0,7 g |
| Na-Salicylat | - | - | - | - |
| Wasserglas | 0,5 g | 0,5 g | 0,7 g | 0,7 g |
| Imidazol | 0,2 g | 0,2 g | 0,1 g | - |
| Turpinal SL | 0,2 g | 0,2 g | - | - |
| Parfum | 0,2 g | 0,2 g | 0,3 g | 0,3 g |
| Weinsäure | ad pH 3,5 | - | ad pH 3,8 | - |
| Monoethanolamin | - | ad pH 9,0 | - | ad pH 9,0 |
| Wasser | ad 100 g | ad 100 g | ad 100 g | ad 100 g |

**Tabelle 2**

| **Komponente** | **A3** | **VA3** | **A4** | **VA4** |
|---|---|---|---|---|
| 4-Hydroxy-2-methoxybenzaldehyd | - | - | - | - |
| 4-Hydroxy-3-methoxybenzaldehyd | 5 mmol | 5 mmol | - | - |
| 3,5-Dimethoxy-4-hydroxybenzaldehyd | 5 mmol | 5 mmol | 5 mmol | 5 mmol |
| 4-Hydroxy-1-naphthaldehyd | - | - | 5 mmol | 5 mmol |
| 4-Formyl-1-methyl-chinolinium-p-toluolsulfonat | - | - | - | - |
| HC Yellow 2 | - | - | - | - |
| Lorol, techn. | 2,5 g | 2,5 g | 1,8 g | 1,8 g |
| Eumulgin B2 | 2,0 g | 2,0 g | 0,8 g | 0,8 g |
| Eumulgin B1 | - | - | - | - |
| Hydrenol D | 7,5 g | 7,5 g | 8,5 g | 8,5 g |
| Texapon NSO | - | - | 15,0 g | 15,0 g |
| Plantacare 1200 | - | - | - | - |
| Plantacare 2000 | - | - | - | - |
| Dehyton K | - | - | 12,5 g | 12,5 g |
| Dehyton AB 30 | - | - | - | - |
| Crodaphos CES | - | - | - | - |
| Crodaphos MCA | - | - | - | - |
| Incroquat DCMC | - | - | 1,5 g | 1,5 g |
| Incroquat Behenyl TMS | - | - | - | - |
| Hydrotriticum QM | 1,0 g | 1,0 g | - | - |
| Abil B 9905 | - | - | - | - |
| Merquat 280 | - | - | - | - |
| Methylparaben | 0,2 g | 0,2 g | - | - |
| Propylparaben | 0,2 g | 0,2 g | - | - |
| Phenoxyethanol | 0,5 g | 0,5 g | 0,8 g | 0,8 g |
| Na-Salicylat | - | - | 0,4 g | 0,4 g |
| Wasserglas | 0,5 g | 0,5 g | 0,9 g | 0,9 g |
| Imidazol | 0,4 g | 0,4 g | - | - |
| Turpinal SL | 0,2 g | 0,2 g | 0,5 g | 0,5 g |
| Parfum | 0,2 g | 0,2 g | 0,1 g | 0,1 g |
| Weinsäure | ad pH 3,6 | - | ad pH 3,9 | - |
| Monoethanolamin | - | ad pH 8,5 | - | ad pH 8,5 |
| Wasser | ad 100 g | ad 100 g | ad 100 g | ad 100 g |

**Tabelle 3**

| **Komponente** | **A5** | **VA5** | **A6** | **VA6** |
|---|---|---|---|---|
| 4-Hydroxy-2-methoxybenzaldehyd | 10 mmol | 10 mmol | 7,5 mmol | 7,5 mmol |
| 4-Hydroxy-3-methoxybenzaldehyd | - | - | - | - |
| 3,5-Dimethoxy-4-hydroxybenzaldehyd | - | - | - | - |
| 4-Hydroxy-1-naphthaldehyd | - | - | - | - |
| 4-Formyl-1-methyl-chinolinium-p- toluolsulfonat | - | - | 2,5 mmol | 2,5 mmol |
| HC Yellow 2 | - | - | 7,5 mmol | 7,5 mmol |
| Lorol, techn. | 2,0 g | 2,0 g | 2,5 g | 2,5 g |
| Eumulgin B2 | 1,0 g | 1,0 g | 2,0 g | 2,0 g |
| Eumulgin B1 | - | - | - | - |
| Hydrenol D | 7,5 g | 7,5 g | 7,5 g | 7,5 g |
| Texapon NSO | - | - | - | - |
| Plantacare 1200 | 4,4 g | 4,4 g | - | - |
| Plantacare 2000 | - | - | 3,5 g | 3,5 g |
| Dehyton K | - | - | - | - |
| Dehyton AB 30 | - | - | 4,0 g | 4,0 g |
| Crodaphos CES | - | - | - | - |
| Crodaphos MCA | - | - | - | - |
| Incroquat DCMC | - | - | - | - |
| Incroquat Behenyl TMS | - | - | - | - |
| Hydrotriticum QM | - | - | - | - |
| Abil B 9905 | 1,0 g | 1,0 g | - | - |
| Merquat 280 | - | - | 2,0 g | 2,0 g |
| Methylparaben | 0,2 g | 0,2 g | 0,3 g | 0,3 g |
| Propylparaben | 0,2 g | 0,2 g | 0,2 g | 0,2 g |
| Phenoxyethanol | 0,5 g | 0,5 g | 0,7 g | 0,7 g |
| Na-Salicylat | - | - | - | - |
| Wasserglas | 0,5 g | 0,5 g | 0,5 g | 0,5 g |
| Imidazol | 0,3 g | 0,3 g | 0,2 g | 0,2 g |
| Turpinal SL | | | 0,2 g | 0,2 g |
| Parfum | 0,3 g | 0,3 g | 0,2 g | 0,2 g |
| Weinsäure | ad pH 4,2 | - | ad pH 3,9 | - |
| Monoethanolamin | - | ad pH 9,0 | - | ad pH 9,5 |
| Wasser | ad 100 g | ad 100 g | ad 100 g | ad 100 g |

**Tabelle 4**

| **Komponente** | **A7** | **VA7** | **A8** | **VA8** |
|---|---|---|---|---|
| 4-Hydroxy-2-methoxybenzaldehyd | 7,5 mmol | 7,5 mmol | - | - |
| 4-Hydroxy-3-methoxybenzaldehyd | - | - | - | - |
| 3,5-Dimethoxy-4- hydroxybenzaldehyd | 7,5 mmol | 7,5 mmol | 10 mmol | 10 mmol |
| 4-Hydroxy-1-naphthaldehyd | - | - | - | - |
| 4-Formyl-1-methyl-chinolinium-p-toluolsulfonat | - | - | - | - |
| HC Yellow 2 | - | - | - | - |
| Lorol, techn. | 3,0 g | 3,0 g | 2,0 g | 2,0 g |
| Eumulgin B2 | 2,0 g | 2,0 g | 1,0 g | 1,0 g |
| Eumulgin B1 | 1,5 g | 1,5 g | 0,4 g | 0,4 g |
| Hydrenol D | 7,0 g | 7,0 g | 8,0 g | 8,0 g |
| Texapon NSO | - | - | - | - |
| Plantacare 1200 | - | - | - | - |
| Plantacare 2000 | - | - | - | - |
| Dehyton K | - | - | 11,5 g | 11,5 g |
| Dehyton AB 30 | - | - | - | - |
| Crodaphos CES | | | 5,0 g | 5,0 g |
| Crodaphos MCA | 3,5 g | 3,5 g | - | - |
| Incroquat DCMC | - | - | - | - |
| Incroquat Behenyl TMS | - | - | - | - |
| Hydrotriticum QM | - | - | - | - |
| Abil B 9905 | - | - | - | - |
| Merquat 280 | - | - | - | - |
| Methylparaben | - | - | 0,2 g | 0,2 g |
| Propylparaben | - | - | 0,2 g | 0,2 g |
| Phenoxyethanol | 0,8 g | 0,8 g | 0,5 g | 0,5 g |
| Na-Salicylat | 0,4 g | 0,4 g | - | - |
| Wasserglas | 0,7 g | 0,7 g | 0,5 g | 0,5 g |
| Imidazol | 0,3 g | 0,3 g | 0,2 g | 0,2 g |
| Turpinal SL | - | - | 0,2 g | 0,2 g |
| Parfum | 0,3 g | 0,3 g | 0,2 g | 0,2 g |
| Weinsäure | ad pH 4,2 | - | ad pH 4,8 | - |
| Monoethanolamin | - | ad pH 9,0 | - | ad pH 8,0 |
| Wasser | ad 100 g | ad 100 g | ad 100 g | ad 100g |

**Tabelle 5**

| **Komponente** | **A9** | **VA9** | **A10** | **VA10** |
|---|---|---|---|---|
| 4-Hydroxy-2-methoxybenzaldehyd | - | - | 10 mmol | 10 mmol |
| 4-Hydroxy-3-methoxybenzaldehyd | - | - | - | - |
| 3,5-Dimethoxy-4- hydroxybenzaldehyd | 10 mmol | 10 mmol | - | - |
| 4-Hydroxy-1-naphthaldehyd | - | - | - | - |
| 4-Formyl-1-methyl-chinolinium-p-toluolsulfonat | - | - | - | - |
| HC Yellow 2 | - | - | - | - |
| Lorol, techn. | 2,0 g | 2,0 g | 2,0 g | 2,0 g |
| Eumulgin B2 | 0,75 g | 0,75 g | 1,0 g | 1,0 g |
| Eumulgin B1 | - | - | 0,4 g | 0,4 g |
| Hydrenol D | - | - | - | - |
| Texapon NSO | 8,5 g | 8,5 g | 8,0 g | 8,0 g |
| Plantacare 1200 | - | - | - | - |
| Plantacare 2000 | 15,0 g | 15,0 g | - | - |
| Dehyton K | - | - | - | - |
| Dehyton AB 30 | - | - | - | - |
| Crodaphos CES | 12,5 g | 12,5 g | 11,5 g | 11,5 g |
| Crodaphos MCA | - | - | - | - |
| Incroquat DCMC | - | - | 5,0 g | 5,0 g |
| Incroquat Behenyl TMS | - | - | - | - |
| Hydrotriticum QM | - | - | | - |
| Abil B 9905 | 0,5 g | 0,5 g | - | - |
| Merquat 280 | - | - | - | - |
| Methylparaben | 0,5 g | 0,5 g | - | - |
| Propylparaben | - | - | - | - |
| Phenoxyethanol | - | - | - | - |
| Na-Salicylat | 0,2 g | 0,2 g | 0,3 g | 0,3 g |
| Wasserglas | 0,2 g | 0,2 g | 0,2 g | 0,2 g |
| Imidazol | 0,5 g | 0,5 g | 0,7 g | 0,7 g |
| Turpinal SL | - | - | - | - |
| Parfum | 0,9 g | 0,9 g | 0,5 g | 0,5 g |
| Weinsäure | ad pH 4,1 | - | ad pH 3,1 | - |
| Monoethanolamin | - | ad pH 8,0 | - | ad pH 9,5 |
| Water, deion. | ad 100 g | ad 100 g | ad 100 g | ad 100 g |

| | |
|---|---|
| Lorol^{®} tech. | C₁₂₋₁₈-Fettalkohol (INCI-Bezeichnung: Coconut alcohol) (Cognis) |
| Eumulgin^{®} B1 | Cetylstearylalkohol mit ca. 12-EO-Einheiten (INCI-Bezeichnung: Ceteareth-12) (Cognis) |
| Eumulgin^{®} B2 | Cetylstearylalkohol mit ca. 20 EO-Einheiten (INCI-Bezeichnung: Ceteareth-20) (Cognis) |
| Hydrenol^{®} D | C₁₆₋₁₈-Fettalkohol (lNCl-Bezeichnung: Cetearyl alcohol) (Cognis) |
| Texapon^{®} NSO | Laurylethersulfat, Natriumsalz (ca. 27,5% Aktivsubstanz; INCI-Bezeichnung: Sodium Laureth Sulfate) (Cognis) |
| Plantacare^{®} 1200 | C₁₂₋₁₆-Fettalkohol-1.4-glucosid (ca. 50-53% Aktivsubstanzgehalt; INCI-Bezeichnung: Lauryl Glucoside, Aqua (Water)) (Cognis) |
| Plantacare^{®} 2000 | C₈₋₁₆ Alkylglucosid (ca. 51-55% Aktivsubstanzgehalt in Wasser; INCI-Bezeichnung: Decyl Glucoside, Aqua (Water)) (Cognis) |
| Dehyton^{®} K | N,N-Dimethyl-N-(C8-18-kokosamidopropyl)ammoniumacetobetain (ca. 30% Aktivsubstanz; INCI-Bezeichnung: Aqua (Water), Cocamidopropyl Betaine) (Cognis) |
| Dehyton AB 30 | Kokosalkyldimethylammoniumbetain, 30% ig; CTFA-Bezeichnung: Coco-Betaine |
| Crodaphos CES | Cetearyl Alcohol (and) Dicetyl Phosphate (and) Ceteth-10 Phosphate, 100% Aktivsubstanz |
| Crodaphos MCA | Cetyl Phosphate, 100 % Aktivsubstanz |
| Incroquat DCMC | Dicetyldimonium Chloride (and) Isopropyl Alcohol, 68% Aktivsubstanz in Isopropanol |
| Incroquat^{®} Behenyl TMC-85 | Trimethylbehenylammoniumchlorid (ca. 77-84% Aktivsubstanzgehalt; INCI-Bezeichnung: Isopropyl Alcohol, Behentrimonium Chloride) (Croda) |
| Hydrotriticum QM | Aqua (and) Cocodimonium Hydroxypropyl Hydrolyzed Wheat Protein, 25% Aktivsubstanz |
| ABIL B 9905 | Polyquaternium-21, 30% Aktivsubstanz |
| Merquat^{®} 280 | Dimethyldiallylammoniumchlorid Acrylsäure Copolymer (ca. 35 Aktivsubstanz in Wasser; INCI-Bezeichnung: Polyquaternium-22) (Ondeo-Nalco) |
| Methylparaben | 4-Hydroxybenzoesäuremethylester |
| Propylparaben | 4-Hydroxybenzoesäurepropylester |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water)) (Solutia) |

### 1.2. Herstellung von Formulierungen des Mittels B

Für das Mittel B wurden aus den in Tabelle 6 bis Tabelle 10 aufgeführten Rezepturbestandteilen die nachfolgenden Formulierungen hergestellt. Bei den Formulierungen handelt es sich sowohl um Gelformulierungen als auch um Cremeformulierungen.

Die dem Stand der Technik entsprechenden Vergleichsformulierungen sind mit VB1 bis VB10 beziffert. Bei diesen Formulierungen wurde durch Zugabe von Monoethanolamin ein alkalischer pH-Wert eingestellt. Die erfindungsgemäßen Formulierungen B1 bis B10 wurden durch Zugabe von Weinsäure auf einen sauren pH-Wert eingestellt.

Die Cremeformulierungen B1, B2, B3, B5 und B6 (sowie die entsprechenden, den Stand der Technik widerspiegelnden Vergleichsformulierungen VB1, VB2, VB3, VB5 und VB6) wurden wie folgt hergestellt: Zunächst wurden Lorol, Eumulgin B1 bzw. Eumulgin B2 und/oder Hydrenol D zusammen bei 80 °C aufgeschmolzen. In diese Schmelze wurden dann bei 80 °C die für die Einzelformulierungen notwendigen Mengenanteile der Komponenten Texapon NSO, Plantacare 1200, Plantacare 2000, Dehyton K, Dehyton AB 30 und/oder Crodaphos CES eingearbeitet. Dieses Gemisch wurde langsam unter Rühren auf Raumtemperatur abkühlen gelassen. Während dieses Abkühlvorganges wurden die übrigen Rezepturbestandteile Wasserglas und/oder Parfum hinzugefügt. Anschließend wurden unter weiterem Rühren die C,H-aciden Verbindungen zugegeben. Dieses sorgfältig vermengte Gemisch wurde mit 80 bis 90 °C heißem Wasser auf 100 g aufgefüllt und erneut unter Rühren abkühlen gelassen. Zum Schluß wurde durch Zugabe von Weinsäure bzw. Monoethanolamin der für die jeweilige Formulierung angegebene pH-Wert eingestellt. Der pH-Wert wurde bei 22 °C gemessen.

Die Gelformulierungen B4, B7, B8, B9 und B10 (sowie die entsprechenden, den Stand der Technik widerspiegelnden Vergleichsformulierungen VB4, VB7, VB8, VB9 und VB10) wurden wie folgt hergestellt:

Die entsprechenden Mengen der C,H-aciden Verbindungen wurden in 70 g Wasser gelöst, dann wurden Wasserglas und/oder Parfum hinzugegeben. Unter Rühren wurden die für die einzelnen Formulierungen notwendigen Anteile an Xanthan Gum und/oder Synthalen CR hinzugefügt, dann wurde mit Wasser auf 100 g aufgefüllt. Der Quellvorgang wurde abgewartet. Zum Schluß wurde durch Zugabe von Weinsäure bzw. Monoethanolamin der für die jeweilige Formulierung angegebene pH-Wert eingestellt. Der pH-Wert wurde bei 22 °C gemessen.

**Tabelle 6**

| **Komponente** | **B1** | **VB1** | **B2** | **VB2** |
|---|---|---|---|---|
| 1,2-Dihydro-1,3,4,6-tetramethyl-2- oxo-pyrimidiniumhydrogensulfat | 10 mmol | 10 mmol | 8 mmol | 8 mmol |
| 1-Allyl-1,2-dihydro-3,4,6-trimethyl- 2-oxopyrimidiniumbromid | - | - | - | - |
| 2-(Cyanmethyl)-benzimidazol | - | - | 2 mmol | 2 mmol |
| Xanthan Gum | - | - | - | - |
| Synthalen CR | - | - | - | - |
| Lorol, techn. | 2,5 g | 2,5 g | 2,0 g | 2,0 g |
| Eumulgin B2 | 0,8 g | 0,8 g | 1,0 g | 1,0 g |
| Eumulgin B1 | | | 0,4 g | 0,4 g |
| Hydrenol D | 8,5 g | 8,5 g | 8,0 g | 8,0 g |
| Texapon NSO | 15,0 g | 15,0 g | - | - |
| Plantacare 1200 | - | - | - | - |
| Plantacare 2000 | - | - | - | - |
| Dehyton K | 11,5 g | 11,5 g | 11,5 g | 11,5 g |
| Dehyton AB 30 | - | - | - | - |
| Crodaphos CES | - | - | 5,0 g | 5,0 g |
| Wasserglas | 0,5 g | 0,5 g | 0,7 g | 0,7 g |
| Parfum | 0,1 g | 0,1 g | 0,2 g | 0,2 g |
| Weinsäure | ad pH 1,0 | - | ad pH 1,2 | - |
| Monoethanolamin | - | ad pH 8,0 | - | ad pH 9,0 |
| Wasser | ad 100 g | ad 100 g | ad 100 g | ad 100 g |

**Tabelle 7**

| **Komponente** | **B3** | **VB3** | **B4** | **VB4** |
|---|---|---|---|---|
| 1,2-Dihydro-1,3,4,6-tetramethyl-2- oxo-pyrimidiniumhydrogensulfat | 10 mmol | 10 mmol | - | - |
| 1-Allyl-1,2-dihydro-3,4,6-trimethyl- 2-oxopyrimidiniumbromid | - | - | 10 mmol | 10 mmol |
| 2-(Cyanmethyl)-benzimidazol | - | - | - | |
| Xanthan Gum | - | - | - | - |
| Synthalen CR | - | - | 3,0 g | 3,0 g |
| Lorol, techn. | 2,5 g | 2,5 g | - | - |
| Eumulgin B2 | 2,0 g | 2,0 g | - | - |
| Eumulgin B1 | - | - | - | - |
| Hydrenol D | 7,5 g | 7,5 g | - | - |
| Wasserglas | - | - | 0,9 g | 0,9 g |
| Parfum | - | - | 0,1 g | 0,1 g |
| Weinsäure | ad pH 1,4 | - | ad pH 1,4 | - |
| Monoethanolamin | - | ad pH 8,5 | - | ad pH 10,0 |
| Wasser | ad 100 g | ad 100 g | ad 100 g | ad 100 g |

**Tabelle 8**

| **Komponente** | **B5** | **VB5** | **B6** | **VB6** |
|---|---|---|---|---|
| 1,2-Dihydro-1,3,4,6-tetramethyl-2- oxo-pyrimidiniumhydrogensulfat | 5 mmol | 5 mmol | 5,6 mmol | 5,6 mmol |
| 1-Allyl-1,2-dihydro-3,4,6-trimethyl- 2-oxopyrimidiniumbromid | 5 mmol | 5 mmol | - | - |
| 2-(Cyanmethyl)-benzimidazol | - | - | 2,4 mmol | 2,4 mmol |
| Xanthan Gum | - | - | - | - |
| Synthalen CR | - | - | - | - |
| Lorol, techn. | 2,0 g | 2,0 g | 2,5 g | 2,5 g |
| Eumulgin B2 | 1,0 g | 1,0 g | 2,0 g | 2,0 g |
| Eumulgin B1 | - | - | - | - |
| Hydrenol D | 7,5 g | 7,5 g | 7,5 g | 7,5 g |
| Texapon NSO | - | - | - | - |
| Plantacare 1200 | 4,4 g | 4,4 g | - | - |
| Plantacare 2000 | - | - | 3,5 g | 3,5 g |
| Dehyton K | - | - | - | - |
| Dehyton AB 30 | - | - | 4,0 g | 4,0 g |
| Crodaphos CES | - | - | - | - |
| Wasserglas | 0,5 g | 0,5 g | - | - |
| Parfum | 0,2 g | 0,2 g | 0,1 g | 0,1 g |
| Weinsäure | ad pH 1,2 | - | ad pH 1,3 | - |
| Monoethanolamin | - | ad pH 8,2 | - | ad pH 8,4 |
| Water. Deion. | ad 100 g | ad 100 g | ad 100 g | ad 100 g |

**Tabelle 9**

| **Komponente** | **B7** | **VB7** | **B8** | **VB8** |
|---|---|---|---|---|
| 1,2-Dihydro-1,3,4,6-tetramethyl-2- oxo-pyrimidiniumhydrogensulfat | 7,5 mmol | 7,5 mmol | - | - |
| 1-Allyl-1,2-dihydro-3,4,6-trimethyl- 2-oxopyrimidiniumbromid | 7,5 mmol | 7,5 mmol | - | - |
| 2-(Cyanmethyl)-benzimidazol | - | - | 10 mmol | 10 mmol |
| Xanthan Gum | - | - | 2,5 g | 2,5 g |
| Synthalen CR | 1,5 g | 1,5 g | 1,0 g | 1,0 g |
| Wasserglas | 0,7 g | 0,7 g | 0,5 g | 0,5 g |
| Parfum | - | - | 0,2 g | 0,2 g |
| Weinsäure | ad pH 1,2 | - | ad pH 1,4 | - |
| Monoethanolamin | - | ad pH 9,2 | - | ad pH 9,4 |
| Wasser | ad 100 g | ad 100 g | ad 100 g | ad 100 g |

**Tabelle 10**

| **Komponente** | **B9** | **VB9** | **B10** | **VB10** |
|---|---|---|---|---|
| 1-Allyl-1,2-dihydro-3,4,6-trimethyl- | | | | |
| 2-oxopyrimidiniumbromid | - | - | 10 mmol | 10 mmol |
| 2-(Cyanmethyl)-benzimidazol | - | - | - | - |
| Xanthan Gum | - | - | 3,5 g | 3,5 g |
| Synthalen CR | 4,5 g | 4,5 g | - | - |
| Wasserglas | 0,9 g | 0,9 g | 0,5 g | 0,5 g |
| Parfum | 0,2 g | 0,2 g | 0,2 g | 0,2 g |
| Weinsäure | ad pH 1,2 | - | ad pH 1,2 | |
| Monoethanolamin | - | ad pH 9,4 | - | ad pH 9,4 |
| Water. Deion. | ad 100 g | ad 100 g | ad 100 g | ad 100 g |

| | |
|---|---|
| Xanthan Gum | Mikrobielles, anionisches Polysaccharid, das D-Glucose, D-Mannose und D-Glucuronsäure, Acetat und Pyrovat im molaren Verhältnis 28:30:20:17:5,1-6,3 enthält |
| Synthalen^{®} CR | 2-(Trimethylammonio)ethylmethacrylatchloridhomopolymer (INCI-Bezeichnung: Polyquaternium-37) (3V Sigma) |
| Lorol^{®} tech. | C₁₂₋₁₈-Fettalkohol (INCI-Bezeichnung: Coconut alcohol) (Cognis) |
| Eumulgin^{®} B1 | Cetylstearylalkohol mit ca. 12-EO-Einheiten (INCI-Bezeichnung: Ceteareth-12) (Cognis) |
| Eumulgin^{®} B2 | Cetylstearylalkohol mit ca. 20 EO-Einheiten (INCI-Bezeichnung: Ceteareth-20) (Cognis) |
| Hydrenol^{®} D | C₁₆₋₁₈-Fettalkohol (INCI-Bezeichnung: Cetearyl alcohol) (Cognis) |
| Texapon^{®} NSO | Laurylethersulfat, Natriumsalz (ca. 27,5% Aktivsubstanz; INCI-Bezeichnung: Sodium Laureth Sulfate) (Cognis) |
| Plantacare^{®} 1200 | C₁₂₋₁₆-Fettalkohol-1.4-glucosid (ca. 50-53% Aktivsubstanzgehalt; INCI-Bezeichnung: Lauryl Glucoside, Aqua (Water)) (Cognis) |
| Plantacare^{®} 2000 | C₈₋₁₆ Alkylglucosid (ca. 51-55% Aktivsubstanzgehalt in Wasser; INCI-Bezeichnung: Decyl Glucoside, Aqua (Water)) (Cognis) |
| Dehyton^{®} K | N,N-Dimethyl-N-(C8-18-kokosamidopropyl)ammoniumacetobetain (ca. 30% Aktivsubstanz; INCI-Bezeichnung: Aqua (Water), Cocamidopropyl Betaine) (Cognis) |
| Dehyton AB 30 | Kokosalkyldimethylammoniumbetain, 30% Aktivsubstanz; CTFA-Bezeichnung: Coco-Betaine |
| Crodaphos CES | Cetearyl Alcohol (and) Dicetyl Phosphate (and) Ceteth-10 Phosphate, 100% Aktivsubstanz |

### 2.1. Lagerung der Formulierungen

Die Formulierungen des Mittels A, des Mittels B und die Vergleichsformulierungen VA sowie VB des Standes der Technik wurden in lichtundurchlässige Glasflaschen abgefüllt und für 4 Wochen bei Raumtemperatur gelagert.

### 3.1. Ausfärbungen

Zur Herstellung der gebrauchsfertigen Färbemischung wurden die bei erfindungsgemäßem pH-Wert gelagerten Formulierungen A und B innig miteinander vermischt. Ebenso wurden die bei nichterfindungsgemäßem, basischem pH-Wert gelagerten Formulierungen VA und VB miteinandert gemischt. Hierbei wurden jeweils die Formulierungen mit derselben Ziffer miteinander vermischt (z.B. A1 wurde mit B1 vermischt, VA1 wurde mit VB1 vermischt usw.).

Direkt anschließend wurde der pH-Wert der zu vergleichenden Mischungen auf den gleichen pH-Wert eingestellt. Zu diesem Zweck wurden diesen Gemischen das Mittel C (siehe Tabellen 11 bis 15) hinzugefügt, welches in der erfindungsgemäßen Form ein Alkalisierungsmittel in einem wässrigen Träger enthält. Dabei wurde zu den Durch das Hinzufügen dieses dritten Mittels wurde der pH-Wert bei 22 °C auf die in den Tabellen 11 bis 15 angegebenen pH-Werte gebracht.

**Tabelle 11**

| **Mittel C** | **Mischung A1/B1** | **Mischung VA1/VB1** | **Mischung A2/B2** | **Mischung VA2/VB2** |
|---|---|---|---|---|
| Mittel C1: 20 Gew.-% Monoethanolamin in Wasser | ad pH 9,5 | ad pH 9,5 | - | - |
| Mittel C2: 10 Gew.-%ige Natriumhydroxidlösung in Wasser | - | - | ad pH 9,5 | ad pH 9,5 |

**Tabelle 12**

| **Mittel C** | **Mischung A3/B3** | **Mischung VA3NB3** | **Mischung A4/B4** | **Mischung VA4NB4** |
|---|---|---|---|---|
| Mittel C3: 10 Gew.-%ige Kaliumhydroxidlösung in Wasser | ad pH 9,0 | ad pH 9,0 | - | - |
| Mittel C4: konzentrierte Ammoniaklösung in Wasser | - | - | ad pH 9,5 | ad pH 9,5 |

**Tabelle 13**

| **Mittel C** | **C5** | **VC5** | **C6** | **VC6** |
|---|---|---|---|---|
| Mittel C1: 20 Gew.-% Monoethanolamin in Wasser | - | - | ad pH 8,9 | ad pH 8,9 |
| **Mittel C5: 10 Gew.-%ige** Natriumcarbonatlösung in Wasser | ad pH 9,0 | ad pH 9,0 | - | - |

**Tabelle 14**

| **Mittel C** | **C7** | **VC7** | **C8** | **VC8** |
|---|---|---|---|---|
| Mittel C2: 10 Gew.-%ige Natriumhydroxidlösung in Wasser | ad pH 9,2 | ad pH 9,2 | - | - |
| Mittel C3: 10 Gew.-%ige Kaliumhydroxidlösung in Wasser | - | - | ad pH 9,4 | ad pH 9,4 |

**Tabelle 15**

| **Mittel C** | **C9** | **VC9** | **C10** | **VC10** |
|---|---|---|---|---|
| Mittel C4: konzentrierte Ammoniaklösung in Wasser | ad pH 9,6 | ad pH 9,6 | - | - |
| Mittel C5: 10 Gew.-%ige Natriumcarbonatlösung in Wasser | - | - | ad pH 9,7 | ad pH 9,7 |

Das erhaltene gebrauchsfertige Färbemittel wurde auf eine Haarsträhne (Kerling naturweiß) aufgebracht (Flottenverhältis-Gewichtsverhältnis: anwendungsbereite Färbemischung zu Gewicht der Haare = 2 : 1) und mit einer Aplizette gleichmäßig verteilt. Nach einer Einwirkzeit von 30 Minuten bei 32 °C wurde die Strähne mit lauwarmem Wasser ausgespült und danach im warmen Luftstrom (30 bis 40 °C) getrocknet. Die Färbungen wurden visuell unter einer Tageslichtlampe beurteilt. Es wurden die folgenden, in Tabelle 16 aufgelisteten Nuancen erhalten.

**Tabelle 16**

| **Mittel** | **Färbung der Haarsträhne** | **Intensität der Färbung** |
|---|---|---|
| A1 + B1 + C1 | dunkelbraun | +++ |
| VA1 + VB1 + C1 | schwach braun | + |
| A2 + B2 + C2 | dunkelbraun | +++ |
| VA2 + VB2 + C2 | braun | + |
| A3 + B3 + C3 | leuchtend blauviolett | +++ |
| VA3 + VB3 + C3 | blass violett | kaum Färbung |
| A4 + B4 + C4 | intensiv dunkelblau | +++ |
| VA4 + VB4 + C4 | blass blau | + |
| A5 + B5 + C1 | leuchtend rot | +++ |
| VA5 + VB5 + C1 | blass rot | + |
| A6 + B6 + C5 | intensiv rotbraun | +++ |
| VA6 + VB6 + C5 | braun | + |
| A7 + B7 + C2 | brillant violett | +++ |
| VA7 + VB7 + C2 | schwach violett | keine Färbung |
| A8 + B8 + C3 | intensiv, orangestichig gelb | +++ |
| VA8 + VB8 + C3 | hellgelb | + |
| A9 + B9 + C4 | intensiv schwarzblau | +++ |
| VA9 + VB9 + C4 | schwach hellblau | + |
| A10 + B10 + C5 | intensiv dunkelrot | +++ |
| VA10 + VB10 + C5 | schwach hellrot | + |

| | | |
|---|---|---|
| Farbintensität: +++ = hoch ++ = mittel + = niedrig | | |

### 4.1. Messung der L*a*b*-Werte ausgewählter Haarsträhnen

Nach dem Färbevorgang wurden ausgewählte Haarsträhnen exemplarisch mit einem Farbmessgerät der Firma Datacolor, Typ Spectraflash 450 farbmetrisch vermessen. Die im Zuge der Messungen erhaltenen L*a*b*-Werte definieren sich wie folgt: Der L*-Wert steht für die Helligkeit, während der a-Wert ein Maß für den Rotanteil ist (je größer der a-Wert ist, desto größer ist der Rotanteil). Der b-Wert ist ein Maß für den Blauanteil einer Farbe, wobei der Blauanteil umso größer ist, je negativer der b-Wert ist.

| **Mittel** | **L*** | **a*** | **b*** | **Nuance** |
|---|---|---|---|---|
| A8 + B8 + C3 | 63,15 | 16,59 | 64,41 | intensiv, orangestichig gelb |
| VA8 + VB8 + C3 | 64,30 | 7,17 | 48,41 | hellgelb |
| A9 + B9 + C4 | 19,86 | 3,84 | 0,76 | intensiv schwarzblau |
| VA9 + VB9 + C4 | 30,99 | 1,03 | -10,61 | schwach hellblau |
| A10 + B10 + C5 | 21,48 | 20,78 | 8,57 | intensiv dunkelrot |
| VA10 + VB10 + C5 | 46,42 | 25,09 | 4,94 | schwach hellrot |

Aus den Beispielen läßt sich entnehmen, daß bei Anwendung der erfindungsgemäßen Formulierungen auf dem Haar auch nach längerer Lagerzeit intensive und brillante Färbungen erhalten werden können. Im Gegensatz hierzu lieferten die alkalisch gestellten Vergleichsformulierungen nach mehrwöchiger Lagerzeit Färbungen von viel geringerer Farbintensität, wie sowohl aus den Nuancenbeurteilungen als auch aus den differierenden L*a*b*-Werten deutlich wird.

## Patentansprüche

1. Kit zum Färben von keratinhaltigen Fasern, insbesondere menschlichen Haaren, umfassend kosmetische Mittel A und B, **dadurch gekennzeichnet, dass**
- das Mittel A in einem kosmetischen Träger mindestens eine reaktive Carbonylverbindung enthält und einen pH-Wert von 2 bis 5 aufweist und
- das Mittel B in einem kosmetischen Träger mindestens eine C,H-acide Verbindung ausgewählt aus Verbindungen der Formel (CH-1) und/oder Verbindungen der Formel (CH-2) enthält, 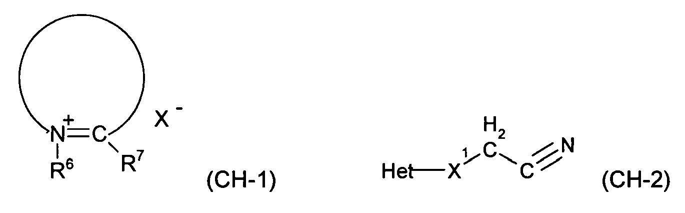 worin
• R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe
R^{I}R^{II}N-(CH₂)ₘ-, worin R^{I} und R^{II} stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
• R⁷ steht für eine (C₁ bis C₆)-Alkylgruppe, insbesondere für eine Methylgruppe,
• X- steht für ein physiologisch verträgliches Anion,
• der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
• Het steht für einen gegebenenfalls substituierten Heteroaromaten,
• X¹ steht für eine direkte Bindung oder eine Carbonylgruppe,
und das Mittel B einen pH-Wert von 0,5 bis 2,5 aufweist.

2. Kit nach Anspruch 1, **dadurch gekennzeichnet, dass** das Mittel A einen pH-Wert von 3 bis 4,5 aufweist.

3. Kit nach einem der Ansprüche 1 oder 2, **dadurch gekennzeichnet, dass** das Mittel B einen pH-Wert von 1 bis 2, insbesondere bevorzugt einen pH-Wert von 1,1 bis 1,9 aufweist.

4. Kit nach einem der Ansprüche 1 bis 3, **dadurch gekennzeichnet, dass** dieser zusätzlich ein Mittel C enthält, **dadurch gekennzeichnet, dass** das Mittel C in einem kosmetischen Träger mindestens ein Alkalisierungsmittel enthält und einen pH-Wert größer als 7 aufweist.

5. Kit nach einem der Ansprüche 1 bis 4, **dadurch gekennzeichnet, dass** die reaktive Carbonylverbindung des Mittels A ausgewählt wird aus mindestens einer Verbindung der Gruppe bestehend aus Benzaldehyd und seinen Derivaten, Naphthaldehyd und seinen Derivaten, Zimtaldehyd und seinen Derivaten, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, Pyridoxal, Antipyrin-4-aldehyd, Furfural, 5-Nitrofurfural, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1 -ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat, -chlorid, -bromid, -iodid, - tetrachlorozinkat, -methylsulfat-, -trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

6. Kit nach einem der Ansprüche 1 bis 5, **dadurch gekennzeichnet, dass** die reaktive Carbonylverbindung des Mittels A ausgewählt wird aus mindestens einer Verbindung der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxybenzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 3-Hydroxy-4-nitrobenzaldehyd, 2-Hydroxy-3-methoxy-5-nitrobenzaldehyd, 5-Nitrovanillin, 2,5-Dinitrosalicylaldehyd, 5-Brom-3-nitrosalicylaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 3-Carboxy-4-hydroxy-benzaldehyd, 5-Carboxyvanillin, 3-Carboxy-4-hydroxy-5-methylbenzaldehyd, 3-Carboxy-5-ethoxy-4-hydroxybenzaldehyd, 3-Carboxy-4-hydroxybenzaldehyd, 5-Carboxyvanillin, 3-Carboxy-4-hydroxy-5-methylbenzaldehyd, 3-Carboxy-5-ethoxy-4-hydroxybenzaldehyd, 3-Allyl-4-hydroxybenzaldehyd, 3-Allyl-4-hydroxy-5-methoxybenzaldehyd, 3-Allyl-4-hydroxy-5-methylbenzaldehyd, 3-Allyl-5-brom-4-hydroxybenzaldehyd, 3,5-Diallyl-4-hydroxybenzaldehyd, 3-Allyl-5-carboxy-4-hydroxybenzaldehyd (3-Allyl-5-formyl-2-hydroxybenzoesäure) und 3-Allyl-4-hydroxy-5-formylbenzaldehyd (5-Allyl-4-hydroxyisophthalaldehyd).

7. Kit nach einem der Ansprüche 1 bis 6, **dadurch gekennzeichnet, dass** die CH-acide Verbindung des Mittels B ausgewählt wird aus mindestens einer Verbindung der Gruppe bestehend aus 2-(2-Furoyl)-acetonitril, 2-(5-Brom-2-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1 H-Benzimidazol-2-ylacetonitril, 1 H-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 2-(6-Hydroxy-4,7-dimethoxy-1-benzofuran-5-oyl)-acetonitril und den Salzen mit physiologisch verträglichem Gegenion X⁻ des 1,2-Dihydro-1,3,4,6-tetramethyl-2-oxo-pyrimidiniums, 1,2-Dihydro-1,3-diethyl-4,6-dimethyl-2-oxo-pyrimidiniums, 1,2-Dihydro-1,3-dipropyl-4,6-dimethyl-2-oxo-pyrimidiniums, 1,2-Dihydro-1,3-di(2-hydroxyethyl)-4,6-dimethyl-2-oxo-pyrimidiniums, 1,2-Dihydro-1,3-diphenyl-4,6-dimethyl-2-oxo-pyrimidiniums, 1,2-Dihydro-1,3,4-trimethyl-2-oxo-pyrimidiniums, 1,2-Dihydro-1,3-diethyl-4-methyl-2-oxo-pyrimidiniums, 1,2-Dihydro-1,3-dipropyl-4-methyl-2-oxo-pyrimidiniums, 1,2-Dihydro-1,3-di(2-hydroxyethyl)-4-methyl-2-oxo-pyrimidiniums, 1,2-Dihydro-1,3-diphenyl-4-methyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums, 1,2-Dihydro-1,3,4,6-tetramethyl-2-thioxo-pyrimidiniums, 1,2-Dihydro-1,3-diethyl-4,6-dimethyl-2-thioxo-pyrimidiniums, 1,2-Dihydro-1,3-dipropyl-4,6-dimethyl-2-thioxo-pyrimidiniums, 1,2-Dihydro-1,3-di(2-hydroxyethyl)-4,6-dimethyl-2-thioxo-pyrimidiniums, 1,2-Dihydro-1,3-diphenyl-4,6-dimethyt-2-thioxo-pyrimidiniums, 1,2-Dihydro-1,3,4-trimethyl-2-thioxo-pyrimidiniums, 1,2-Dihydro-1,3-diethyl-4-methyl-2-thioxo-pyrimidiniums, 1,2-Dihydro-1,3-dipropyl-4-methyl-2-thioxo-pyrimidiniums, 1,2-Dihydro-1,3-di(2-hydroxyethyl)-4-methyl-2-thioxo-pyrimidiniums, 1,2-Dihydro-1,3-diphenyl-4-methyl-2-thioxo-pyrimidiniums, 1,2-Dihydro-3,4-dimethyl-2-oxo-chinazoliniums und 1,2-Dihydro-3,4-dimethyl-2-thioxo-chinazoliniums.

8. Kit nach einem der Ansprüche 1 bis 7, **dadurch gekennzeichnet, dass** die reaktiven Carbonylverbindungen des Mittels A in einer Menge von 0,03 bis 65,00 mmol, insbesondere 1,00 bis 30,00 mmol, bezogen auf 100 g des Mittels A, enthalten sind.

9. Kit nach einem der Ansprüche 1 bis 8, **dadurch gekennzeichnet, dass** die CH-aciden Verbindungen der Formel (CH-1) und/oder (CH-2) in dem Mittel B in einer Menge von 0,03 bis 65,00 mmol, insbesondere 1,00 bis 30,00 mmol, bezogen auf 100 g des Mittels B, enthalten sind.

10. Verwendung eines kosmetischen Trägermediums mit einem pH-Wert von 2 bis 5 zur Stabilisierung von darin enthaltenen reaktiven Carbonylverbindungen.

11. Verwendung eines kosmetischen Trägermediums mit einem pH-Wert von 0,5 bis 2,5 zur Stabilisierung von darin enthaltenen CH-aciden Verbindungen.

12. Verfahren zum Färben von keratinhaltigen Fasern, insbesondere menschlichen Haaren, **dadurch gekennzeichnet dass**
- mindestens ein Mittel A, das in einem kosmetischen Träger mindestens eine reaktive Carbonylverbindung enthält und einen pH-Wert von 2 bis 5 aufweist und
- mindestens ein Mittel B, das in einem kosmetischen Träger mindestens eine C,H-acide Verbindung ausgewählt aus Verbindungen der Formel (CH-1) und/oder Verbindungen der Formel (CH-2) enthält, 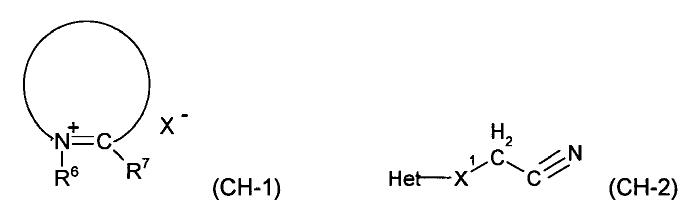 worin
• R⁶ steht für eine lineare oder cyclische (C₁ bis C₆)-Alkylgruppe, eine (C₂ bis C₆)-Alkenylgruppe, eine gegebenenfalls substituierte Arylgruppe, eine gegebenenfalls substituierte Heteroarylgruppe, eine Aryl-(C₁ bis C₆)-alkylgruppe, eine (C₁ bis C₆)-Hydroxyalkylgruppe, eine (C₂ bis C₆)-Polyhydroxyalkylgruppe, eine (C₁ bis C₆)-Alkoxy-(C₁ bis C₆)-alkylgruppe, eine Gruppe
R^{I}R^{II}N-(CH₂)ₘ-, worin R' und R" stehen unabhängig voneinander für ein Wasserstoffatom, eine (C₁ bis C₄)-Alkylgruppe, eine (C₁ bis C₄)-Hydroxyalkylgruppe oder eine Aryl-(C₁ bis C₆)-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
• R⁷ steht für eine (C₁ bis C₆)-Alkylgruppe, insbesondere für eine Methylgruppe,
• X- steht für ein physiologisch verträgliches Anion,
• der Zyklus der Formel (CH-1) repräsentiert alle Ringstrukturen, die zusätzlich weitere Heteroatome wie Stickstoff, Sauerstoff oder Schwefel enthalten können und ferner ankondensierte Ringstrukturen tragen können, wobei alle diese Ringsstrukturen zusätzliche Substituenten tragen können,
• Het steht für einen gegebenenfalls substituierten Heteroaromaten,
• X¹ steht für eine direkte Bindung oder eine Carbonylgruppe,
und einen pH-Wert von 0,5 bis 2,5 aufweist.
miteinander vermischt werden, die resultierende Mischung auf die keratinhaltige Faser aufgebracht, 5 bis 60 Minuten auf der Faser belassen und anschießend wieder ausgespült oder mit einem Shampoo ausgewaschen wird.

13. Verfahren nach Anspruch 12, **dadurch gekennzeichnet, dass** die Mischung aus Mittel A und Mittel B durch Zugabe mindestens eines weiteren Mittels C auf einen alkalischen pH-Wert gebracht wird, wobei das Mittel C in einem kosmetischen Träger mindestens ein Alkalisierungsmittel enthält und einen pH-Wert größer als 7 aufweist.
